# EUROPÄISCHE PATENTANMELDUNG

(11) **EP 3 088 414 A1**
(43) Veröffentlichungstag der Anmeldung: **02.11.2016**
(21) Anmeldenummer: 15001277.1
(22) Anmeldetag: 30.04.2015
(51) Int. Cl.: C07K 14/415, A61K 39/35, C07K 17/14, G01N 33/551, G01N 33/68

(54) **VERBESSERTER NACHWEIS VON NUSSALLERGIEN**

(71) Anmelder: Euroimmun Medizinische Labordiagnostika AG, 23560 Lübeck (DE)
(72) Erfinder: Ehlers, Anna, 23564 Lübeck (DE); Michel, Yvonne, 20146 Hamburg (DE); Rohwer, Stefanie, 23617 Stockelsdorf (DE); Suer, Waltraud, 23911 Buchholz (DE); Unger, Mandy, 20255 Hamburg (DE)

(57) **Zusammenfassung**

Die vorliegende Erfindung betrifft ein Polypeptid umfassend ein antigenes Fragment mit einer Sequenz von wenigstens sieben Aminosäuren aus einer hydrophilen Domäne eines Ölkörperassoziierten Proteins, wobei das Polypeptid keine oder eine verkürzte hydrophobe Domäne des Ölkörper-assoziierten Proteins aufweist, und wobei das Ölkörper-assoziierte Protein aus der Gruppe ausgewählt ist, die Oleosin, Caleosin und Steroleosin umfasst, eine pharmazeutische Zusammensetzung oder einen Impfstoff umfassend das Polypeptid, einen Komplex umfassend das Polypeptid und einen daran gebundenen Antikörper, eine medizinische oder diagnostische Vorrichtung umfassend das Polypeptid, eine Nucleinsäure kodierend das Polypeptid oder eine Zelle umfassend diese Nukleinsäure, einen Testkit umfassend das Polypeptid sowie ein Verfahren zum Nachweisen eines Antikörpers, der an das Polypeptid bindet.

SEQUENCE LISTING

<110> EUROIMMUN

<120> Verbesserter Nachweis von Nussallergien

<130> P00045

<160> 23

<170> BiSSAP 1.3

<210> 1
<211> 32
<212> PRT
<213> Artificial Sequence

<220>
<223> N-terminale hydrophile Domäne des Oleosins aus der Haselnuss

<400> 1
Met Ala Asp Arg Pro Gln Gln Leu Gln Val His Pro Gln Arg Gly His
1               5                   10                  15
Gly His Tyr Glu Gly Gly Ile Lys Asn Gln Arg Gly Gly Gly Pro Ser
            20                  25                  30

<210> 2
<211> 49
<212> PRT
<213> Artificial Sequence

<220>
<223> C-terminale hydrophile Domäne des Oleosins aus der Haselnuss

<400> 2
Asn Tyr Leu Arg Cys Ala Ser Gln Ser Leu Pro Arg Glu Met Asp Gln
1               5                   10                  15
Ala Lys Arg Arg Met Gln Asp Met Ala Ala Phe Val Gly Gln Lys Thr
            20                  25                  30
Arg Glu Val Gly Gln Glu Ile Gln Ser Arg Ala Gln Glu Gly Arg Arg
        35                  40                  45
Thr

<210> 3
<211> 19
<212> PRT
<213> Artificial Sequence

<220>
<223> N-terminale hydrophile Domäne des Oleosins aus der Walnuss

<400> 3
Met Ala Glu His Gln Gln Gln Ser Gln His Pro Ala Gln Gln Pro Arg
1               5                   10                  15
Ser His Gln

<210> 4
<211> 47
<212> PRT
<213> Artificial Sequence

<220>
<223> C-terminale hydrophile Domäne des Oleosins aus der Walnuss

<400> 4
Tyr Arg Tyr Met Thr Gly Arg Gln Pro Pro Gly Thr Glu Gln Ile Asp
1               5                   10                  15
Gln Ala Arg Met Lys Leu Ala Ser Lys Ala Arg Glu Met Lys Asp Lys
            20                  25                  30
Ala Glu Gln Phe Gly Gln Gln His Val Ser Gly Ala Gln Ser Ser
        35                  40                  45

<210> 5
<211> 105
<212> PRT
<213> Artificial Sequence

<220>
<223> Konsensus-Sequenz I von Oleosinen


<400> 5
Xaa Pro Xaa Xaa Xaa Xaa Xaa Xaa Xaa Xaa Xaa Xaa Xaa Xaa Xaa Xaa
1               5                   10                  15
Xaa Xaa Xaa Leu Leu Xaa Xaa Xaa Gly Xaa Xaa Leu Xaa Xaa Xaa Xaa
            20                  25                  30
Xaa Xaa Leu Xaa Xaa Xaa Xaa Pro Xaa Xaa Xaa Xaa Phe Ser Pro Xaa
        35                  40                  45
Xaa Val Pro Ala Xaa Xaa Xaa Xaa Xaa Xaa Xaa Xaa Xaa Xaa Phe Leu
    50                  55                  60
Xaa Ser Gly Xaa Xaa Gly Xaa Xaa Xaa Xaa Xaa Xaa Xaa Xaa Trp Xaa
65                  70                  75                  80
Xaa Xaa Xaa Xaa Xaa Xaa Xaa Xaa Xaa Xaa Xaa Xaa Xaa Xaa Xaa Xaa
                85                  90                  95
Xaa Ala Xaa Xaa Xaa Xaa Xaa Xaa Xaa
            100                 105

<210> 6
<211> 105
<212> PRT
<213> Artificial Sequence

<220>
<223> Konsensus-Sequenz II von Oleosinen

<220>
<221> VARIANT
<222> 1
<223> wherein Xaa is a Gly or a Gln

<220>
<221> VARIANT
<222> 3
<223> wherein Xaa is a Ser or an Arg

<220>
<221> VARIANT
<222> 9
<223> wherein Xaa is a Lys or an Ala

<220>
<221> VARIANT
<222> 12
<223> wherein Xaa is an Ala or a Thr

<220>
<221> VARIANT
<222> 17
<223> wherein Xaa is a Gly or a Ser

<220>
<221> VARIANT

<222> 18
<223> wherein Xaa is an Ala or a Gly

<220>
<221> VARIANT
<222> 24
<223> wherein Xaa is an Ala or a Ser

<220>
<221> VARIANT
<222> 26
<223> wherein Xaa is a Leu or an Ile

<220>
<221> VARIANT
<222> 30
<223> wherein Xaa is a Ser or a Gly

<220>
<221> VARIANT
<222> 31
<223> wherein Xaa is a Thr or a Ser

<220>
<221> VARIANT
<222> 33
<223> wherein Xaa is a Leu or an Ile

<220>
<221> VARIANT
<222> 39
<223> wherein Xaa is Thr or a Ser

<220>
<221> VARIANT
<222> 41
<223> wherein Xaa is a Leu or a Val

<220>
<221> VARIANT
<222> 42
<223> wherein Xaa is a Phe or a Leu

<220>
<221> VARIANT
<222> 43
<223> wherein Xaa is a Val or an Ile

<220>
<221> VARIANT
<222> 44
<223> wherein Xaa is an Ile or a Leu

<220>
<221> VARIANT
<222> 48
<223> wherein Xaa is an Ile or a Val

<220>
<221> VARIANT
<222> 49
<223> wherein Xaa is a Leu or an Ile

<220>
<221> VARIANT
<222> 54
<223> wherein Xaa is a Leu or an Ile

<220>
<221> VARIANT
<222> 56
<223> wherein Xaa is an Ile or a Val

<220>
<221> VARIANT
<222> 58
<223> wherein Xaa is a Leu or Gly

<220>
<221> VARIANT
<222> 68
<223> wherein Xaa is a Gly or an Ala

<220>
<221> VARIANT
<222> 72
<223> wherein Xaa is a Thr or an Ala

<220>
<221> VARIANT
<222> 73
<223> wherein Xaa is an Ala or a Gly

<220>
<221> VARIANT
<222> 75
<223> wherein Xaa is a Ser or a Thr

<220>
<221> VARIANT
<222> 76
<223> wherein Xaa is a Ser or a Val

<220>
<221> VARIANT
<222> 78
<223> wherein Xaa is a Ser or a Thr

<220>
<221> VARIANT
<222> 82
<223> wherein Xaa is an Asn or an Arg

<220>
<221> VARIANT
<222> 83
<223> wherein Xaa is a Tyr or a Ser

<220>
<221> VARIANT
<222> 85
<223> wherein Xaa is an Arg or a Thr

<220>
<221> VARIANT
<222> 94
<223> wherein Xaa is a Gln or a Glu

<220>
<221> VARIANT
<222> 96
<223> wherein Xaa is an Asp or a Glu

<220>
<221> VARIANT
<222> 99

<223> wherein Xaa is an Arg or a Lys

<400> 6
Xaa Pro Xaa Xaa Xaa Xaa Xaa Xaa Xaa Xaa Xaa Xaa Xaa Xaa Xaa Xaa
1               5                   10                  15
Xaa Xaa Xaa Leu Leu Xaa Xaa Xaa Gly Xaa Xaa Leu Xaa Xaa Xaa Xaa
            20                  25                  30
Xaa Xaa Leu Xaa Xaa Xaa Xaa Pro Xaa Xaa Xaa Xaa Phe Ser Pro Xaa
        35                  40                  45
Xaa Val Pro Ala Xaa Xaa Xaa Xaa Xaa Xaa Xaa Xaa Xaa Xaa Phe Leu
    50                  55                  60
Xaa Ser Gly Xaa Xaa Gly Xaa Xaa Xaa Xaa Xaa Xaa Xaa Xaa Trp Xaa
65                  70                  75                  80
Xaa Xaa Xaa Xaa Xaa Xaa Xaa Xaa Xaa Xaa Xaa Xaa Xaa Xaa Xaa Xaa
                85                  90                  95
Xaa Ala Xaa Xaa Xaa Xaa Xaa Xaa Xaa
            100                 105

<210> 7
<211> 10
<212> PRT
<213> Artificial Sequence

<220>
<223> C-terminales Sequenzmotiv I von Oleosinen

<400> 7
Leu Xaa Xaa Lys Ala Arg Glu Xaa Lys Asp
1               5                   10

<210> 8
<211> 6
<212> PRT
<213> Artificial Sequence

<220>
<223> C-terminales Sequenzmotiv II von Oleosinen

<220>
<221> VARIANT
<222> 3
<223> wherein Xaa is a Gln or a Glu

<220>
<221> VARIANT
<222> 6
<223> wherein Xaa is a Lys or an Arg

<400> 8
Val Gly Xaa Lys Thr Xaa
1               5

<210> 9
<211> 12
<212> PRT
<213> Artificial Sequence

<220>
<223> Konsensus-Sequenz I für hydrophobe Domäne von Oleosinen

<400> 9
Pro Xaa Xaa Xaa Xaa Phe Ser Pro Xaa Xaa Val Pro
1               5                   10

<210> 10
<211> 12
<212> PRT
<213> Artificial Sequence

<220>
<223> Konsensus-Sequenz II für hydrophobe Domäne von Oleosinen

<220>
<221> VARIANT
<222> 2
<223> wherein Xaa is a Leucin or a Val

<220>
<221> VARIANT
<222> 3
<223> wherein Xaa is a Phe or a Leu

<220>
<221> VARIANT
<222> 4
<223> wherein Xaa is a Val or an Ile

<220>
<221> VARIANT
<222> 5
<223> wherein Xaa is a Leu or an Ile

<220>
<221> VARIANT
<222> 9
<223> wherein Xaa is an Ile or a Val

<220>
<221> VARIANT
<222> 10
<223> wherein Xaa is a Leu or an Ile

<400> 10
Pro Xaa Xaa Xaa Xaa Phe Ser Pro Xaa Xaa Val Pro
1               5                   10

<210> 11
<211> 235
<212> PRT
<213> Artificial Sequence

<220>
<223> Konsensus-Sequenz I von Caleosinen

<400> 11
   Xaa Xaa Xaa Xaa Xaa Xaa Xaa Xaa Xaa Xaa Xaa Xaa Xaa Xaa Xaa Xaa
   1               5                   10                  15
   Xaa Xaa Xaa Ala Xaa Xaa Ala Xaa Xaa Thr Xaa Xaa Arg Xaa Xaa Arg
               20                  25                  30
   Xaa Asp Leu Xaa Xaa Xaa Xaa Pro Lys Pro Tyr Xaa Xaa Arg Xaa Xaa
           35                  40                  45
   Xaa Ala Pro Asp Xaa Xaa Xaa Xaa Xaa Gly Thr Xaa Xaa His Xaa Xaa
       50                  55                  60
   Xaa Xaa Xaa Ser Val Leu Gln Gln His Xaa Xaa Phe Phe Asp Xaa Asp
   65                  70                  75                  80
   Xaa Xaa Gly Ile Ile Tyr Pro Xaa Glu Thr Xaa Xaa Gly Xaa Arg Xaa
                   85                  90                  95
   Xaa Gly Phe Asn Xaa Xaa Xaa Ser Xaa Ile Xaa Xaa Xaa Xaa Xaa Xaa
               100                 105                 110
   Xaa Xaa Xaa Ser Tyr Xaa Thr Leu Pro Xaa Trp Xaa Pro Ser Xaa Xaa
           115                 120                 125
   Phe Xaa Ile Xaa Xaa Xaa Asn Ile His Xaa Xaa Lys His Gly Ser Asp
       130                 135                 140
   Ser Xaa Xaa Tyr Asp Xaa Glu Gly Arg Xaa Xaa Pro Xaa Asn Xaa Glu
   145                 150                 155                 160
   Xaa Xaa Phe Ser Lys Xaa Ala Xaa Thr Xaa Pro Asp Xaa Leu Xaa Xaa
   165                         170                         175
   Xaa Glu Leu Trp Xaa Xaa Thr Xaa Xaa Asn Arg Xaa Ala Xaa Asp Xaa
               180                 185                 190
   Phe Gly Trp Xaa Ala Xaa Lys Xaa Glu Trp Xaa Xaa Leu Tyr Xaa Leu
           195                 200                 205
   Ala Xaa Asp Xaa Xaa Gly Xaa Leu Xaa Lys Glu Ala Xaa Arg Arg Cys
       210                 215                 220
   Xaa Asp Gly Ser Leu Phe Glu Tyr Cys Ala Lys
   225                 230                 235

<210> 12
<211> 235
<212> PRT
<213> Artificial Sequence

<220>
<223> Konsensus-Sequenz II von Caleosinen

<220>
<221> VARIANT
<222> 15
<223> wherein Xaa is an Ala or a Ser

<220>
<221> VARIANT
<222> 16
<223> wherein Xaa is a Met or a Leu

<220>
<221> VARIANT
<222> 18
<223> wherein Xaa is a Pro or a Thr

<220>
<221> VARIANT
<222> 21
<223> wherein Xaa is a Pro or a Glu

<220>
<221> VARIANT
<222> 24
<223> wherein Xaa is a Pro or an Arg

<220>
<221> VARIANT
<222> 25
<223> wherein Xaa is a Val or an Ile

<220>
<221> VARIANT
<222> 31
<223> wherein Xaa is a Val or an Ala

<220>
<221> VARIANT
<222> 36
<223> wherein Xaa is a Glu or an Asp

<220>
<221> VARIANT
<222> 39
<223> wherein Xaa is an Ile or a Leu

<220>
<221> VARIANT
<222> 44
<223> wherein Xaa is a Leu or a Met

<220>
<221> VARIANT
<222> 45
<223> wherein Xaa is a Pro or an Ala

<220>
<221> VARIANT
<222> 47
<223> wherein Xaa is a Gly or an Ala

<220>
<221> VARIANT
<222> 48
<223> wherein Xaa is a Leu or a Met

<220>
<221> VARIANT
<222> 55
<223> wherein Xaa is a His or an Asn

<220>
<221> VARIANT
<222> 56
<223> wherein Xaa is a Pro or an Ile

<220>
<221> VARIANT
<222> 61
<223> wherein Xaa is a Gly or an Asp

<220>
<221> VARIANT
<222> 64
<223> wherein Xaa is a His or an Asn

<220>
<221> VARIANT
<222> 66
<223> wherein Xaa is a Gly or an Asn

<220>
<221> VARIANT
<222> 67
<223> wherein Xaa is a Leu or a Met

<220>
<221> VARIANT
<222> 75
<223> wherein Xaa is an Ala or a Ser

<220>
<221> VARIANT
<222> 82
<223> wherein Xaa is an Asn or an Asp

<220>
<221> VARIANT
<222> 91
<223> wherein Xaa is a Tyr or a Phe

<220>
<221> VARIANT
<222> 94
<223> wherein Xaa is a Leu or a Phe

<220>
<221> VARIANT
<222> 97

<223> wherein Xaa is an Ile or a Leu

<220>
<221> VARIANT
<222> 103
<223> wherein Xaa is an Ala or a Gly

<220>
<221> VARIANT
<222> 108
<223> wherein Xaa is an Ala or a Met

<220>
<221> VARIANT
<222> 111
<223> wherein Xaa is an Ile or a Val

<220>
<221> VARIANT
<222> 112
<223> wherein Xaa is an Asn or a His

<220>
<221> VARIANT
<222> 115
<223> wherein Xaa is a Leu or a Met

<220>
<221> VARIANT
<222> 118
<223> wherein Xaa is a Pro or an Ala

<220>
<221> VARIANT
<222> 127
<223> wherein Xaa is a Pro or a Leu

<220>
<221> VARIANT
<222> 130
<223> wherein Xaa is a Pro or an Ala

<220>
<221> VARIANT
<222> 132
<223> wherein Xaa is a Tyr or a His

<220>
<221> VARIANT
<222> 133
<223> wherein Xaa is a Leu or an Ile

<220>
<221> VARIANT
<222> 138
<223> wherein Xaa is a Lys or an Arg

<220>
<221> VARIANT
<222> 139
<223> wherein Xaa is an Ala or a Ser

<220>
<221> VARIANT
<222> 146
<223> wherein Xaa is a Gly or a Lys

<220>

<221> VARIANT
<222> 150
<223> wherein Xaa is a Thr or a an Asn

<220>
<221> VARIANT
<222> 154
<223> wherein Xaa is a Tyr or a Phe

<220>
<221> VARIANT
<222> 161
<223> wherein Xaa is an Asn or a Leu

<220>
<221> VARIANT
<222> 166
<223> wherein Xaa is a His or a Tyr

<220>
<221> VARIANT
<222> 173
<223> wherein Xaa is an Arg or a Lys

<220>
<221> VARIANT
<222> 175
<223> wherein Xaa is a Thr or a Ser

<220>
<221> VARIANT
<222> 176
<223> wherein Xaa is a Leu or Phe

<220>
<221> VARIANT
<222> 177
<223> wherein Xaa is a Gly or a Lys

<220>
<221> VARIANT
<222> 182
<223> wherein Xaa is a Met or a Lys

<220>
<221> VARIANT
<222> 184
<223> wherein Xaa is a Glu or a Gln

<220>
<221> VARIANT
<222> 185
<223> wherein Xaa is an Ala or a Gly

<220>
<221> VARIANT
<222> 190
<223> wherein Xaa is a Phe or a Trp

<220>
<221> VARIANT
<222> 192
<223> wherein Xaa is a Phe or an Ile

<220>
<221> VARIANT
<222> 196
<223> wherein Xaa is an Ile or a Leu

<220>
<221> VARIANT
<222> 203
<223> wherein Xaa is a Thr or a Gly

<220>
<221> VARIANT
<222> 204
<223> wherein Xaa is a Leu or a Val

<220>
<221> VARIANT
<222> 212
<223> wherein Xaa is a Gln or a Glu

<220>
<221> VARIANT
<222> 215
<223> wherein Xaa is a Phe or a His

<220>
<221> VARIANT
<222> 217
<223> wherein Xaa is a Ser or an Ala

<220>
<221> VARIANT
<222> 221
<223> wherein Xaa is an Ile or a Val

<220>
<221> VARIANT
<222> 225
<223> wherein Xaa is a Tyr or a Phe

<400> 12
Xaa Xaa Xaa Xaa Xaa Xaa Xaa Xaa Xaa Xaa Xaa Xaa Xaa Xaa Xaa Xaa
1               5                   10                  15
Xaa Xaa Xaa Ala Xaa Xaa Ala Xaa Xaa Thr Xaa Xaa Arg Xaa Xaa Arg
            20                  25                  30
Xaa Asp Leu Xaa Xaa Xaa Xaa Pro Lys Pro Tyr Xaa Xaa Arg Xaa Xaa
        35                  40                  45
Xaa Ala Pro Asp Xaa Xaa Xaa Xaa Xaa Gly Thr Xaa Xaa His Xaa Xaa
    50                  55                  60
Xaa Xaa Xaa Ser Val Leu Gln Gln His Xaa Xaa Phe Phe Asp Xaa Asp
65                  70                  75                  80
Xaa Xaa Gly Ile Ile Tyr Pro Xaa Glu Thr Xaa Xaa Gly Xaa Arg Xaa
                85                  90                  95
Xaa Gly Phe Asn Xaa Xaa Xaa Ser Xaa Ile Xaa Xaa Xaa Xaa Xaa Xaa
            100                 105                 110
Xaa Xaa Xaa Ser Tyr Xaa Thr Leu Pro Xaa Trp Xaa Pro Ser Xaa Xaa
        115                 120                 125
Phe Xaa Ile Xaa Xaa Xaa Asn Ile His Xaa Xaa Lys His Gly Ser Asp
    130                 135                 140
Ser Xaa Xaa Tyr Asp Xaa Glu Gly Arg Xaa Xaa Pro Xaa Asn Xaa Glu
145                 150                 155                 160
Xaa Xaa Phe Ser Lys Xaa Ala Xaa Thr Xaa Pro Asp Xaa Leu Xaa Xaa
                165                 170                 175
Xaa Glu Leu Trp Xaa Xaa Thr Xaa Xaa Asn Arg Xaa Ala Xaa Asp Xaa
            180                 185                 190
Phe Gly Trp Xaa Ala Xaa Lys Xaa Glu Trp Xaa Xaa Leu Tyr Xaa Leu
        195                 200                 205
Ala Xaa Asp Xaa Xaa Gly Xaa Leu Xaa Lys Glu Ala Xaa Arg Arg Cys
    210                 215                 220
Xaa Asp Gly Ser Leu Phe Glu Tyr Cys Ala Lys
225                 230                 235

<210> 13
<211> 11
<212> PRT
<213> Artificial Sequence

<220>
<223> Konsensus-Sequenz I für hydrophobe Domäne von Caleosinen

<400> 13
Pro Xaa Trp Xaa Pro Ser Xaa Xaa Phe Xaa Ile
1               5                   10

<210> 14
<211> 10
<212> PRT
<213> Artificial Sequence

<220>
<223> Konsensus-Sequenz II für hydrophobe Domäne von Caleosinen

<220>
<221> VARIANT
<222> 7
<223> wherein Xaa is a Pro or a Leu

<220>
<221> VARIANT
<222> 10
<223> wherein Xaa is a Prolin or an Ala

<400> 14
Pro Xaa Trp Xaa Pro Ser Xaa Xaa Phe Xaa
1               5                   10

<210> 15
<211> 242
<212> PRT
<213> Artificial Sequence

<220>
<223> Konsensus-Sequenz I von Steroleosinen

<400> 15
Xaa Leu Xaa Xaa Xaa Xaa Xaa Asn Xaa Xaa Xaa Pro Xaa Xaa Xaa Xaa
1               5                   10                  15
Xaa Xaa Xaa Xaa Xaa Xaa Xaa Pro Xaa Xaa Xaa Xaa Xaa Xaa Xaa Xaa
            20                  25                  30
Xaa Xaa Xaa Xaa Xaa Xaa Xaa Xaa Xaa Xaa Xaa Xaa Xaa Xaa Xaa Lys
        35                  40                  45
Val Val Xaa Ile Thr Gly Ala Ser Xaa Xaa Ile Gly Glu Xaa Xaa Ala
    50                  55                  60
Tyr Xaa Tyr Ala Xaa Xaa Xaa Ala Xaa Leu Xaa Leu Xaa Ala Xaa Arg
65                  70                  75                  80
Xaa Xaa Xaa Leu Xaa Xaa Xaa Xaa Xaa Xaa Ala Arg Xaa Xaa Gly Xaa
                85                  90                  95
Xaa Xaa Val Xaa Xaa Xaa Xaa Ala Asp Val Xaa Lys Xaa Xaa Xaa Cys
            100                 105                 110
Xaa Xaa Xaa Xaa Xaa Xaa Xaa Xaa Xaa Xaa Xaa Gly Arg Xaa Asp His
        115                 120                 125
Leu Val Asn Xaa Xaa Xaa Xaa Xaa Xaa Xaa Xaa Xaa Xaa Xaa Xaa Xaa
    130                 135                 140
Xaa Xaa Xaa Xaa Xaa Xaa Xaa Xaa Xaa Xaa Xaa Xaa Asn Phe Trp Gly
145                 150                 155                 160
Xaa Xaa Tyr Xaa Thr Xaa Xaa Ala Xaa Xaa Xaa Leu Xaa Xaa Xaa Xaa
                165                 170                 175
Gly Xaa Xaa Xaa Xaa Xaa Xaa Ser Xaa Xaa Xaa Trp Xaa Xaa Xaa Pro
            180                 185                 190
Arg Xaa Xaa Xaa Tyr Xaa Ala Xaa Lys Xaa Ala Xaa Xaa Xaa Xaa Xaa

195                     200                     205
Glu Thr Leu Arg Xaa Glu Xaa Xaa Xaa Xaa Xaa Xaa Xaa Thr Xaa Xaa
    210                     215                     220
Thr Xaa Gly Xaa Xaa Xaa Xaa Glu Xaa Xaa Xaa Gly Lys Xaa Xaa Xaa
225                     230                     235                     240
Xaa Xaa

<210> 16
<211> 242
<212> PRT
<213> Artificial Sequence

<220>
<223> Konsensus-Sequenz II von Steroleosinen

<220>
<221> VARIANT
<222> 1
<223> wherein Xaa is a Glu or an Asp

<220>
<221> VARIANT
<222> 3
<223> wherein Xaa is a Leu or an Ile

<220>
<221> VARIANT
<222> 4
<223> wherein Xaa is a His or an Asn

<220>
<221> VARIANT
<222> 7
<223> wherein Xaa is a Leu or a Met

<220>
<221> VARIANT
<222> 9
<223> wherein Xaa is a Leu or a Phe

<220>
<221> VARIANT
<222> 11
<223> wherein Xaa is an Ala or a Val

<220>
<221> VARIANT
<222> 13
<223> wherein Xaa is a Phe or a Pro

<220>
<221> VARIANT
<222> 14
<223> wherein Xaa is a Phe or an Ala

<220>
<221> VARIANT
<222> 15
<223> wherein Xaa is a Ser or a Thr

<220>
<221> VARIANT
<222> 19
<223> wherein Xaa is a Met or a Leu

<220>
<221> VARIANT

<222> 21
<223> wherein Xaa is a Phe or a Leu

<220>
<221> VARIANT
<222> 23
<223> wherein Xaa is a Leu or a Trp

<220>
<221> VARIANT
<222> 35
<223> wherein Xaa is an Ile or a Leu

<220>
<221> VARIANT
<222> 43
<223> wherein Xaa is an Asp or a Glu

<220>
<221> VARIANT
<222> 44
<223> wherein Xaa is an Asn or an Asp

<220>
<221> VARIANT
<222> 57
<223> wherein Xaa is a Ser or a Pro

<220>
<221> VARIANT
<222> 58
<223> wherein Xaa is a Gly or an Ala

<220>
<221> VARIANT
<222> 63
<223> wherein Xaa is an Ile or a Leu

<220>
<221> VARIANT
<222> 66
<223> wherein Xaa is a Glu or a Gln

<220>
<221> VARIANT
<222> 70
<223> wherein Xaa is an Arg or a Lys

<220>
<221> VARIANT
<222> 71
<223> wherein Xaa is a Gly or an Arg

<220>
<221> VARIANT
<222> 73
<223> wherein Xaa is an Asn or a Cys

<220>
<221> VARIANT
<222> 75
<223> wherein Xaa is a Val or an Ala

<220>
<221> VARIANT
<222> 79
<223> wherein Xaa is an Arg or a Leu

<220>
<221> VARIANT
<222> 81
<223> wherein Xaa is a Glu or a Lys

<220>
<221> VARIANT
<222> 87
<223> wherein Xaa is an Ile or a Val

<220>
<221> VARIANT
<222> 88
<223> wherein Xaa is a Ser or an Ala

<220>
<221> VARIANT
<222> 96
<223> wherein Xaa is a Ser or an Ala

<220>
<221> VARIANT
<222> 107
<223> wherein Xaa is a Val or a Ser

<220>
<221> VARIANT
<222> 110
<223> wherein Xaa is a Glu or an Asp

<220>
<221> VARIANT
<222> 111
<223> wherein Xaa is a Glu or an Asp

<220>
<221> VARIANT
<222> 113
<223> wherein Xaa is an Arg or a Met

<220>
<221> VARIANT
<222> 114
<223> wherein Xaa is an Arg or a Lys

<220>
<221> VARIANT
<222> 116
<223> wherein Xaa is an Ile or a Val

<220>
<221> VARIANT
<222> 118
<223> wherein Xaa is a Glu or a Gln

<220>
<221> VARIANT
<222> 119
<223> wherein Xaa is a Thr or an Ala

<220>
<221> VARIANT
<222> 121
<223> wherein Xaa is an Asn or a Ser

<220>
<221> VARIANT
<222> 123

<223> wherein Xaa is a Tyr or a Phe

<220>
<221> VARIANT
<222> 126
<223> wherein Xaa is a Val or a Leu

<220>
<221> VARIANT
<222> 133
<223> wherein Xaa is a Val or an Ala

<220>
<221> VARIANT
<222> 135
<223> wherein Xaa is a Leu or an Ile

<220>
<221> VARIANT
<222> 141
<223> wherein Xaa is a Phe or a Leu

<220>
<221> VARIANT
<222> 142
<223> wherein Xaa is a Glu or an Asp

<220>
<221> VARIANT
<222> 143
<223> wherein Xaa is a Glu or an Asn

<220>
<221> VARIANT
<222> 146
<223> wherein Xaa is an Asp or a Glu

<220>
<221> VARIANT
<222> 148
<223> wherein Xaa is a Ser or a Thr

<220>
<221> VARIANT
<222> 151
<223> wherein Xaa is a Pro or an Arg

<220>
<221> VARIANT
<222> 154
<223> wherein Xaa is a Met or a Leu

<220>
<221> VARIANT
<222> 155
<223> wherein Xaa is an Asp or a Glu

<220>
<221> VARIANT
<222> 156
<223> wherein Xaa is an Ile or a Thr

<220>
<221> VARIANT
<222> 162
<223> wherein Xaa is a Val or an Ile

<220>

<221> VARIANT
<222> 169
<223> wherein Xaa is a Leu or an Ala

<220>
<221> VARIANT
<222> 170
<223> wherein Xaa is a Pro or a Gln

<220>
<221> VARIANT
<222> 171
<223> wherein Xaa is a Tyr or a His

<220>
<221> VARIANT
<222> 175
<223> wherein Xaa is a Ser or a Thr

<220>
<221> VARIANT
<222> 176
<223> wherein Xaa is an Asn or an Arg

<220>
<221> VARIANT
<222> 178
<223> wherein Xaa is an Arg or a Lys

<220>
<221> VARIANT
<222> 179
<223> wherein Xaa is an Ile or a Val

<220>
<221> VARIANT
<222> 180
<223> wherein Xaa is an Ile or a Val

<220>
<221> VARIANT
<222> 183
<223> wherein Xaa is an Ala or a Ser

<220>
<221> VARIANT
<222> 185
<223> wherein Xaa is a Val or a Ser

<220>
<221> VARIANT
<222> 189
<223> wherein Xaa is a Leu or a Met

<220>
<221> VARIANT
<222> 190
<223> wherein Xaa is a Pro or a Thr

<220>
<221> VARIANT
<222> 194
<223> wherein Xaa is a Met or an Arg

<220>
<221> VARIANT
<222> 195
<223> wherein Xaa is a Ser or a His

<220>
<221> VARIANT
<222> 200
<223> wherein Xaa is an Ala or a Ser

<220>
<221> VARIANT
<222> 202
<223> wherein Xaa is a Ser or an Ala

<220>
<221> VARIANT
<222> 204
<223> wherein Xaa is a Leu or an Ile

<220>
<221> VARIANT
<222> 207
<223> wherein Xaa is a Phe or a Leu

<220>
<221> VARIANT
<222> 208
<223> wherein Xaa is a Tyr or a Phe

<220>
<221> VARIANT
<222> 218
<223> wherein Xaa is a Glu or an Asp

<220>
<221> VARIANT
<222> 219
<223> wherein Xaa is a Val or an Ile

<220>
<221> VARIANT
<222> 220
<223> wherein Xaa is a Gly or a His

<220>
<221> VARIANT
<222> 221
<223> wherein Xaa is an Ile or a Val

<220>
<221> VARIANT
<222> 223
<223> wherein Xaa is an Ile or a Val

<220>
<221> VARIANT
<222> 224
<223> wherein Xaa is a Val or an Ala

<220>
<221> VARIANT
<222> 226
<223> wherein Xaa is a His or a Pro

<220>
<221> VARIANT
<222> 229
<223> wherein Xaa is an Ile or a Thr

<220>
<221> VARIANT

<222> 238
<223> wherein Xaa is a Phe or a Tyr

<400> 16
Xaa Leu Xaa Xaa Xaa Xaa Xaa Asn Xaa Xaa Xaa Pro Xaa Xaa Xaa Xaa
1               5                   10                  15
Xaa Xaa Xaa Xaa Xaa Xaa Xaa Pro Xaa Xaa Xaa Xaa Xaa Xaa Xaa Xaa
            20                  25                  30
Xaa Xaa Xaa Xaa Xaa Xaa Xaa Xaa Xaa Xaa Xaa Xaa Xaa Xaa Xaa Lys
        35                  40                  45
Val Val Xaa Ile Thr Gly Ala Ser Xaa Xaa Ile Gly Glu Xaa Xaa Ala
    50                  55                  60
Tyr Xaa Tyr Ala Xaa Xaa Xaa Ala Xaa Leu Xaa Leu Xaa Ala Xaa Arg
65                  70                  75                  80
Xaa Xaa Xaa Leu Xaa Xaa Xaa Xaa Xaa Xaa Ala Arg Xaa Xaa Gly Xaa
                85                  90                  95
Xaa Xaa Val Xaa Xaa Xaa Xaa Ala Asp Val Xaa Lys Xaa Xaa Xaa Cys
            100                 105                 110
Xaa Xaa Xaa Xaa Xaa Xaa Xaa Xaa Xaa Xaa Xaa Gly Arg Xaa Asp His
        115                 120                 125
Leu Val Asn Xaa Xaa Xaa Xaa Xaa Xaa Xaa Xaa Xaa Xaa Xaa Xaa Xaa
    130                 135                 140
Xaa Xaa Xaa Xaa Xaa Xaa Xaa Xaa Xaa Xaa Xaa Xaa Asn Phe Trp Gly
145                 150                 155                 160
Xaa Xaa Tyr Xaa Thr Xaa Xaa Ala Xaa Xaa Xaa Leu Xaa Xaa Xaa Xaa
                165                 170                 175
Gly Xaa Xaa Xaa Xaa Xaa Xaa Ser Xaa Xaa Xaa Trp Xaa Xaa Xaa Pro
            180                 185                 190
Arg Xaa Xaa Xaa Tyr Xaa Ala Xaa Lys Xaa Ala Xaa Xaa Xaa Xaa Xaa
        195                 200                 205
Glu Thr Leu Arg Xaa Glu Xaa Xaa Xaa Xaa Xaa Xaa Thr Xaa Xaa
    210                 215                 220
Thr Xaa Gly Xaa Xaa Xaa Xaa Glu Xaa Xaa Xaa Gly Lys Xaa Xaa Xaa
225                 230                 235                 240
Xaa Xaa


<210> 17
<211> 13
<212> PRT
<213> Artificial Sequence

<220>
<223> Konsensus-Sequenz I für hydrophobe Domäne von Steroleosinen

<220>
<221> VARIANT
<222> 2
<223> wherein Xaa is a Pro or a Phe

<220>
<221> VARIANT
<222> 3
<223> wherein Xaa is a Phe or an Ala

<220>
<221> VARIANT
<222> 4
<223> wherein Xaa is a Ser or a Thr

<220>
<221> VARIANT
<222> 8
<223> wherein Xaa is a Met or a Leu

<220>
<221> VARIANT
<222> 10

<223> wherein Xaa is a Phe or a Leu

<220>
<221> VARIANT
<222> 12
<223> wherein Xaa is a Leu or a Trp

<400> 17
Pro Xaa Xaa Xaa Xaa Xaa Xaa Xaa Xaa Xaa Xaa Xaa Pro
1               5                   10

<210> 18
<211> 12
<212> PRT
<213> Artificial Sequence

<220>
<223> C-terminales Sequenzmotiv I von Steroleosinen

<220>
<221> VARIANT
<222> 1
<223> wherein Xaa is a Lys or a Gln

<220>
<221> VARIANT
<222> 2
<223> wherein Xaa is a Glu or an Asp

<220>
<221> VARIANT
<222> 5
<223> wherein Xaa is a Glu or an Asp

<220>
<221> VARIANT
<222> 6
<223> wherein Xaa is a Val or a Met

<400> 18
Xaa Xaa Xaa Arg Xaa Xaa Xaa Xaa Xaa Xaa Xaa Pro
1               5                   10

<210> 19
<211> 46
<212> PRT
<213> Artificial Sequence

<220>
<223> C-terminales Sequenzmotiv II von Steroleosinen

<220>
<221> VARIANT
<222> 1
<223> wherein Xaa is a Val or a Leu

<220>
<221> VARIANT
<222> 2
<223> wherein Xaa is a Glu or a Ser

<220>
<221> VARIANT
<222> 8
<223> wherein Xaa is an Ile or a Met

<220>
<221> VARIANT

<222> 9
<223> wherein Xaa is a Val or an Ile

<220>
<221> VARIANT
<222> 11
<223> wherein Xaa is a Gly or a Ser

<220>
<221> VARIANT
<222> 15
<223> wherein Xaa is a Gly or a Lys

<220>
<221> VARIANT
<222> 18
<223> wherein Xaa is a Tyr or a Cys

<220>
<221> VARIANT
<222> 19
<223> wherein Xaa is a Val or a Leu

<220>
<221> VARIANT
<222> 20
<223> wherein Xaa is a Lys or a Thr

<220>
<221> VARIANT
<222> 25
<223> wherein Xaa is a Tyr or a Phe

<220>
<221> VARIANT
<222> 28
<223> wherein Xaa is a Phe or a Thr

<220>
<221> VARIANT
<222> 30
<223> wherein Xaa is a Leu or a Trp

<220>
<221> VARIANT
<222> 32
<223> wherein Xaa is an Arg or a Lys

<220>
<221> VARIANT
<222> 33
<223> wherein Xaa is a Val or Leu

<220>
<221> VARIANT
<222> 34
<223> wherein Xaa is a Phe or a Leu

<220>
<221> VARIANT
<222> 38
<223> wherein Xaa is a Val or a Leu

<220>
<221> VARIANT
<222> 43
<223> wherein Xaa is a Phe or a Cys

<220>
<221> VARIANT
<222> 44
<223> wherein Xaa is an Arg or a Lys

<220>
<221> VARIANT
<222> 45
<223> wherein Xaa is a Phe or a Leu

<220>
<221> VARIANT
<222> 46
<223> wherein Xaa is a Leu or a Met

<400> 19
Xaa Xaa Xaa Xaa Ala Xaa Xaa Xaa Xaa Xaa Xaa Xaa Xaa Arg Xaa Xaa
1               5                   10                  15
Xaa Xaa Xaa Xaa Xaa Pro Xaa Trp Xaa Xaa Xaa Xaa Xaa Xaa Xaa Xaa
            20                  25                  30
Xaa Xaa Xaa Pro Xaa Xaa Xaa Xaa Trp Xaa Xaa Xaa Xaa Xaa
        35                  40                  45

<210> 20
<211> 15
<212> PRT
<213> Artificial Sequence

<220>
<223> C-terminales Sequenzmotiv III von Steroleosinen

<220>
<221> VARIANT
<222> 3
<223> wherein Xaa is an Ile or a Val

<220>
<221> VARIANT
<222> 7
<223> wherein Xaa is a Pro or a Thr

<220>
<221> VARIANT
<222> 9
<223> wherein Xaa is a Val or an Ala

<220>
<221> VARIANT
<222> 10
<223> wherein Xaa is a Lys or an Arg

<220>
<221> VARIANT
<222> 13
<223> wherein Xaa is a Phe or a Leu

<400> 20
Lys Lys Xaa Leu Asp Xaa Xaa Gly Xaa Xaa Xaa Xaa Xaa Tyr Pro
1               5                   10                  15

<210> 21
<211> 13
<212> PRT
<213> Artificial Sequence

<220>
<223> C-terminales Sequenzmotiv IV von Steroleosinen

<220>
<221> VARIANT
<222> 1
<223> wherein Xaa is a Phe or an Arg

<220>
<221> VARIANT
<222> 4
<223> wherein Xaa is a Leu or an Ile

<220>
<221> VARIANT
<222> 10
<223> wherein Xaa is an Arg or a Lys

<400> 21
Xaa Xaa Leu Xaa Xaa Xaa Xaa Gly Xaa Xaa Xaa Xaa Ser
1               5                   10

<210> 22
<211> 6
<212> PRT
<213> Artificial Sequence

<220>
<223> Beispielhafte Linkersequenz

<400> 22
Thr Leu Ala Gly Ser Val
1               5

<210> 23
<211> 87
<212> PRT
<213> Artificial Sequence

<220>
<223> Beispielhaftes Konstrukt abgeleitet von Cor a

<400> 23
Met Ala Asp Arg Pro Gln Gln Leu Gln Val His Pro Gln Arg Gly His
1               5                   10                  15
Gly His Tyr Glu Gly Gly Ile Lys Asn Gln Arg Gly Gly Gly Pro Ser
            20                  25                  30
Thr Leu Ala Gly Ser Val Asn Tyr Leu Arg Cys Ala Ser Gln Ser Leu
        35                  40                  45
Pro Arg Glu Met Asp Gln Ala Lys Arg Arg Met Gln Asp Met Ala Ala
    50                  55                  60
Phe Val Gly Gln Lys Thr Arg Glu Val Gly Gln Glu Ile Gln Ser Arg
65                  70                  75                  80
Ala Gln Glu Gly Arg Arg Thr
                85

## Beschreibung

Die vorliegende Erfindung betrifft ein Polypeptid umfassend ein antigenes Fragment mit einer Sequenz von wenigstens sieben Aminosäuren aus einer hydrophilen Domäne eines Ölkörperassoziierten Proteins, wobei das Polypeptid keine oder eine verkürzte hydrophobe Domäne des Ölkörper-assoziierten Proteins aufweist, und wobei das Ölkörper-assoziierte Protein aus der Gruppe ausgewählt ist, die Oleosin, Caleosin und Steroleosin umfasst, eine pharmazeutische Zusammensetzung oder einen Impfstoff umfassend das Polypeptid, einen Komplex umfassend das Polypeptid und einen daran gebundenen Antikörper, eine medizinische oder diagnostische Vorrichtung umfassend das Polypeptid, eine Nucleinsäure kodierend das Polypeptid oder eine Zelle umfassend diese Nucleinsäure, einen Testkit umfassend das Polypeptid sowie eine Verfahren zum Nachweisen eines Antikörpers, der gegen das Polypeptid bindet.

Neben Milch, Eiern, Soja, Weizen, Fisch, Schalentieren und Erdnüssen gehören Samen und Kerne zu den "Big 8" der Allergenquellen, die für bis zu 90 % der allergischen Reaktionen gegen Nahrungsmittel verantwortlich sind. Mit einem jährlichen Importvolumen von 200 Megatonnen sind die Hasel- und Walnuss die relevantesten Nusssorten in Deutschland (Hanson, R. und A. Medina, 2012, Tree nuts annual 2012, USDA Foreign Agriculture Service).

Eine allergische Reaktion ist eine Überempfindlichkeit des Immunsystems gegen eine nichtpathogene Substanz aus der Umwelt, wodurch klinische Krankheitssymptome ausgebildet werden (Pirquet, C. von 1906, "Allergie", Münchener medizinische Wochenschrift 53, S. 1457-8). Besonders in den hochentwickelten Industriestaaten sind mit einem Viertel der Bevölkerung immer mehr Menschen gegen ein oder mehrere Allergene sensibilisiert (Galli, S. J., M. Tsai und A. M. Piliponsky 2008, "The development of allergic inflammation", Nature 454.7203, S. 445-54). Die auftretenden Symptome reichen von Urtikaria über Erbrechen und Diarrhoe bis hin zum anaphylaktischen Schock.

Für die eindeutige Diagnose einer Nussallergie ist die Entwicklung eines Komponentenspezifischen in vitro-Allergietests von großem Interesse. Durch den Einsatz von Einzelallergenen können Primärsensibilisierungen und Kreuzreaktivitäten identifiziert werden, wodurch ein Sensibilisierungsmuster erstellt wird. Durch solch ein Muster kann eine verbesserte Risikoabschätzung erfolgen, die zu einem optimierten Therapieansatz führt (Treudler, R. 2012, "In-vitro-Allergiediagnostik aktuell", Journal of the German Society of Dermatology 2.10, S. 89-100).

Die Haselnuss, *Corylus avellana,* gehört zur Familie der Birkengewächse (*Betulaceae*). Es konnte von Zuidmeer-Jongejan et al. ("Oil body-associated hazelnut allergens including oleosins are underrepresented in diagnostic extracts but associated with servere symptoms", Clinical and Translational Allergy 4.1, S. 1 -10) nachgewiesen werden, dass in Allergenextrakten aufgrund des Herstellungsprozesses die als Allergene identifizierten Oleosine (Cor a 12 und Cor a 13) unterrepräsentiert sind. Oleosine stehen im Verdacht, starke systemische Reaktionen bis hin zum anaphylaktischen Schock auszulösen (Kleine-Tebbe, J. et al., 2009, in-vitro-Diagnostik und molekulare Grundlagen von IgE-vermittelten Nahrungsmittelallergien, 18.01.2015, Gesellschaft für Pädiatrische Allergologie und Umweltmedizin e. V). Bislang wurden die Oleosine aus den Nüssen noch wenig untersucht, da ihre Isolierung durch den langen hydrophoben Abschnitt eine große Herausforderung darstellt (Hsieh, K. und A. H. Huang 2004, "Endoplasmic Reticulum, Oleosins, and Oils in Seeds and Tapetum Cells", Plant Physiology 136.3, S. 3427-34). In kommerziellen Tests werden bis heute noch keine Ölkörper-assoziierten Nuss-Allergene eingesetzt.

Die englische Walnuss, lat. *Juglans regia,* gehört wie die Pekannuss zur Familie der Walnussgewächse (lat. Juglandaceae) und ist die bevorzugt konsumierte Walnussart in Nordamerika (Roux, K. H., S. S. Teuber und S. K. Sathe (2003). "Tree nut Allergens". International Archives of Allergy and Immunology 131.4, S. 234-44). Im Jahre 2012 betrug der Konsum innerhalb der Europäischen Union (EU) 239,5 Megatonnen (Hanson, R. und A. Medina (2012). Tree nuts annual 2012. USDA Foreign Agriculture Service.). Identifizierte Majorallergene der Walnuss sind ein 2S Albumin (Jug r 1), ein Vicilin (Jug r 2), ein LTP (Jug r 3) sowie ein 11S-Globulin (Jug r 4). Eine Sensibilisierung gegen Majorallergene der Walnuss kann zu schweren systemischen Reaktionen führen, so dass durch den Verzehr ein anaphylaktischer Schock ausgelöst werden kann (Agreiter, C. (2013). "Purification of seed storage proteins from tree nuts and peanut and their range of cross-reactivity - implication for the nut allergic patient". Magisterarb. University of Wien.). Aufgrund einer 75 %igen Homologie zwischen Jug r 2 und Ara h 1 (Vicilin der Erdnuss) kann eine Sensibilisierung gegen Jug r 2 zu einer Kreuzreaktivität gegen Erdnüsse führen. Kreuzreaktivitäten zwischen Walnuss und Pfirsich treten ebenfalls auf, da das LTP Jug r 3 eine hohe Homologie zu dem LTP aus dem Pfirsich aufweist (Roux, K. H., S. S. Teuber und S. K. Sathe (2003). 3Tree nut Allergens". International Archives of Allergy and Immunology 131.4, S. 234 -44).

Die Pekannuss, *Carya illinoinensis,* gehört zur Familie der Walnussgewächse. Im Jahr 2013 wurden schätzungsweise 440 Millionen Pfund Pekannüsse in den USA verzehrt (Nature's Finest Food, Ltd., 2013, US Domestic Pecan Consumption Rises. 07.02.2015). Sharma identifizierte 2010 die Allergene Car i 1, Car i 2 und Car i 4 und konnte diese Allergene rekombinant darstellen. Für die Allergene Car i 1, ein 2S-Albumin, und Car i 4, ein 11S-Globulin, wurden Epitope identifiziert, die verdauungsresistent sind. Beim 2S-Albumin Car i 1 konnten fünf stark reaktive, lineare Epitope identifiziert werden, die möglicherweise die Ursache dafür sind, dass Car i 1 starke allergische Reaktionen auslösen kann (Sharma G. M. et al., 2011, "Cloning and characterization of 2S albumin, Car i 1, a major allergen in pecan". Journal of Agricultural and Food Chemistry 59.8, S. 4130-9). Das Allergen Car i 4 verfügt ebenfalls über stark reaktive, lineare Epitope, die auf der sauren Untereinheit des 11S-Globulins liegen (Sharma G. M. et al., 2011 b, "Cloning and Characterization of an 11 S Legumin, Car i 4, a Major Allergen in Pecan", Journal of Agricultural and Food Chemistry 59.17, S. 9542-52). Durch Inhibitionstests konnte nachgewiesen werden, dass die spezifischen IgE-Antikörper gegen die Walnuss mit der Pekannuss kreuzreagieren (Teuber, S. S., S. K. Sathe, K. H. Roux und W. R. Petersen, 2000, "Cross-reactivity of walnut and other tree nuts by IgE immunoblot inhibition", Journal of Allergy and Clinical Immunology 105.1, S. 140).

Eine Allergie kann unter Verwendung verschiedener Testprinzipien diagnostiziert werden. Neben der Anamnese, bei der ein Arzt durch gezielte Fragen Informationen über den Patienten sammelt und Hinweise auf mögliche Allergieauslöser erfragt, werden meist mehrere Untersuchungsverfahren in Kombination zur Aufklärung hinzugezogen. Dabei kommen in vivo Hauttests und in vitro-Labortests zum Einsatz. Beim in vivo-Skin-Prick-Test z.B. werden Allergenextrakte oder auch Einzelallergene auf die Haut des Patienten aufgetragen und die Oberhaut durch den Tropfen hindurch leicht angeritzt. Eine positive Reaktion lässt sich durch die Bildung von Quaddeln erkennen (Sicherer et al., 2009, "Allergie Frontiers: Diagnosis and Health Economics.", Springer Verlag).

In vitro können durch den Nachweis allergenspezifischer Antikörper im Blut des Patienten Informationen auf molekularer Ebene gesammelt werden (Renz et al., 2010, "In-vitro-Allergiediagnostik - Leitlinie der Deutschen Gesellschaft für Allergologie und klinische Immunologie (DGAKI) unter Beteiligung des Ärzteverbandes Deutscher Allergologen (ÄDA), der Gesellschaft für Pädiatrische Allergologie und Umweltmedizin (GPA) und der Deutschen Dermatologischen Gesellschaft (DDG)", Allergo Journal, 19, S. 110-28). Für den Nachweis einer Sensibilisierung des Patienten werden dabei Allergenextrakte oder Einzelallergene auf einer Festphase immobilisiert und anschließend mit dem Patientenserum inkubiert. An das Allergen gebundene spezifische Antikörper können zum Nachweis mit einem markierten sekundären Antikörper detektiert werden. (Lutmann et al., 2009, "Der Experimentator-Immunologie", Spektrum Akademischer Verlag). In den vergangenen Jahren hat sich die Verwendung von rekombinant hergestellten und nativen, isolierten Einzelallergenen in serologischen Tests durchgesetzt. Das als Komponenten-spezifische Diagnostik bezeichnete Verfahren ermöglicht eine differenziertere Aussage zum Sensibilisierungsmuster bei polyvalenter Allergie (Hemmer, W. (2009). "Allergiediagnostik - Extrakte oder Moleküle?" Wiener Medizinische Wochenschrift 6.8, S. 9-11).

Als weitere in vitro-Verfahren sind zelluläre Tests zu nennen, bei denen in einem funktionellen, zellulären System allergische Reaktionen untersucht werden. Dazu gehören z.B. die Bestimmung der in vitro-Histaminfreisetzung aus peripheren Leukozyten, die Degranulation der basophilen Leukozyten, der Basophilenaktivierungstest und der der Lymphozyten-Transformations-Test (Ring, J. In: "Angewandte Allergologie", Urban & Vogel, München, 2004, 3. Auflage).

Als Goldstandard der Allergiediagnostik gilt der Provokationstest, mit dem eine eindeutige Diagnose gestellt werden kann. Dem Patienten wird dabei eine immer höhere Allergendosis zugeführt, bisher Symptome zeigt (Bock et al., "Double-blind, placebo-controlled food challenge (DBPCFC) as an office procedure: a manual.", Journal of Allergy and Clinical Immunology, 1988; 82.6, S. 986-97, Niggemann et al, "Standardisierung von oralen Provokationstests bei Verdacht auf Nahrungsmittelallergie", Allergo Journal 2011; 20:149-60,Riechelmann et al, 2002, "Durchführung des nasalen Provokationstests bei Erkrankungen der oberen Atemwege", Allergo Journal 2002; 11:29-36.).

Während zahlreiche Standardantigene routinemäßig zum Nachweis von allergieindikativen IgE eingesetzt werden, bedauert die Fachwelt, dass Ölkörper-assoziierte Proteine beim Aufbau routinemäßiger Tests bisher nicht ausreichend berücksichtigt werden konnten.

Gründe dafür umfassen zunächst die schlechte Isolierbarkeit und geringe Stabilität solcher Proteine, insbesondere bei fordernden klimatischen Bedingungen, die herkömmlich als Volllängen-Proteine aus Ölkörper-Fraktionen von Nüssen isoliert werden (Zuidmeer-Jongehan et al., 2014). Zusätzlich können auf diesem Wege immer nur geringe Mengen in Chargen schlecht reproduzierbarer Qualität erhalten werden. Im Stand der Technik sind keine Verfahren beschrieben, die eine Isolierung von ausreichend stabilem Antigen im Großmaßstab gestatten.

Mit Hinblick auf die Qualität von Tests, die mit derartigen Reagenzien aufgebaut werden, zeigt sich die Fachwelt unzufrieden. Ausdrücklich wird die geringe Empfindlichkeit und Unzuverlässigkeit beklagt (Zuidmeer-Jongehan et al., 2014).

Ein weiteres Problem besteht darin, dass die kommerziell erhältlichen Tests keine belastbare Aussage gestatten, welchen Schweregrad die Allergie eines Patienten aufweist, beispielweise, ob ein Patient nur unter einer Allergie mit leichten Symptomen leidet oder es zu schweren, bis zu lebensbedrohlichen Komplikationen kommen kann.

Vor diesem Hintergrund besteht ein erheblicher Bedarf an neuen Reagenzien und Verfahren auf Basis von Ölkörper-assoziierten Proteinen zum Nachweis von Lebensmittelallergien, besonders gegen Pflanzenteil wie Samen und Kerne, genauer Nüsse.

Der vorliegenden Erfindung liegt die Aufgabe zu Grunde, die genannten und weitere Nachteile der im Stand der Technik beschriebenen Reagenzien und Verfahren zur Diagnose von Allergien gegen Nüsse zu überwinden.

Eine weitere Aufgabe besteht darin, mit Hinblick auf Spezifität und/oder Sensitivität vorteilhafte Reagenzien und Verfahren zur Diagnose von Allergien gegen Nüsse bereitzustellen. Insbesondere soll der Anteil an falsch-positiven und/oder falsch-negativen Ergebnissen verringert werden.

Eine weitere Aufgabe besteht darin, industriell im routinemäßig und/oder Großmaßstab herstellbare Reagenzien und zur Herstellung geeignete Verfahren bereitzustellen, sowie Tests zum Nachweis von Nussallergien mit reproduzierbaren Eigenschaften, die im routinemäßig und/oder Großmaßstab hergestellt werden können.

Eine weitere Aufgabe besteht darin, möglichst unempfindliche Reagenzien bereitzustellen, die unter möglichst extremen klimatischen Bedingungen zuverlässig funktionieren.

Eine weitere Aufgabe besteht darin, einen Test, Reagenzien und Verfahren bereitzustellen, die eine differenzierte Diagnose bzw. Prognose von Allergien gestatten, insbesondere eine Einschätzung des Schweregrades der Allergie, unter der ein Patient leidet.

Diese und weitere Aufgaben werden durch den Gegenstand der vorliegenden Anmeldung und insbesondere auch durch den Gegenstand der beigefügten unabhängigen Ansprüche gelöst, wobei sich Ausführungsformen aus den Unteransprüchen ergeben.

Die der Erfindung zu Grunde liegende Aufgabe wird in einem ersten Aspekt gelöst durch ein Polypeptid umfassend ein antigenes Fragment mit einer Sequenz von wenigstens sieben Aminosäuren aus einer hydrophilen Domäne eines Ölkörper-assoziierten Proteins, wobei das Polypeptid keine oder eine verkürzte hydrophobe Domäne des Ölkörper-assoziierten Proteins aufweist, und wobei das Ölkörper-assoziierte Protein aus der Gruppe ausgewählt ist, die Oleosin, Caleosin und Steroleosin umfasst.

In einer ersten bevorzugten Ausführungsform des ersten Aspekts umfasst das Polypeptid
a) ein antigenes Fragment von wenigstens sieben Aminosäuren aus einer N-terminalen hydrophilen Domäne eines Ölkörper-assoziierten Proteins und
b) ein antigenes Fragment von wenigstens sieben Aminosäuren aus einer C-terminalen hydrophilen Domäne eines Ölkörper-assoziierten Proteins,
wobei a) und b) aneinander fusioniert oder durch einen Linker verbunden sind, und wobei das Ölkörper-assoziierte Protein aus der Gruppe ausgewählt ist, die Oleosin und Caleosin umfasst.

In einer zweiten bevorzugten Ausführungsform des ersten Aspekts, bei der es sich auch um eine Ausführungsform der ersten Ausführungsform handelt, ist das Ölkörper-assoziierte Protein ein Oleosin.

In einer dritten bevorzugten Ausführungsform, bei der es sich auch um eine Ausführungsform der ersten bis zweiten Ausführungsform handelt, handelt es sich bei a) um eine Sequenz aus SEQ ID NO 1 und Varianten davon und bei b) um eine Sequenz aus SEQ ID NO 2 und Varianten davon.

In einer vierten bevorzugten Ausführungsform, bei der es sich auch um eine Ausführungsform der ersten bis dritten Ausführungsform handelt, handelt es sich bei a) um eine Sequenz aus SEQ ID NO 3 und Varianten davon und bei b) um eine Sequenz aus SEQ ID NO 4 und Varianten davon.

In der fünften bevorzugten Ausführungsform, bei der es sich auch um eine Ausführungsform der ersten bis fünften Ausführungsform handelt, ist das Polypeptid immobilisiert, bevorzugt an einem festen Träger, noch bevorzugter einem Mikrokügelchen.

In einem zweiten Aspekt wird das Problem gelöst durch eine pharmazeutische Zusammensetzung oder einen Impfstoff umfassend das Polypeptid.

In einem dritten Aspekt wird das Problem gelöst durch einen Komplex umfassend das Polypeptid sowie einen daran gebundenen Antikörper, bevorzugt der IgE-Klasse.

In einem vierten Aspekt wird das Problem gelöst durch eine medizinische oder diagnostische Vorrichtung umfassend das erfindungsgemäße Polypeptid.

In einer bevorzugten Ausführungsform des vierten Aspekts handelt es sich um einen Teststreifen, einen Biochip oder ein Elektrophoresegel.

In einem fünften Aspekt wird das Problem gelöst durch eine Nucleinsäure kodierend für das erfindungsgemäße Polypeptid oder die Zelle umfassend diese Nucleinsäure.

In einem sechsten Aspekt wird das Problem gelöst durch ein Testkit umfassend das erfindungsgemäße Polypeptid, optional zusätzlich umfassend ein Mittel zum Nachweis des erfindungsgemäßen Komplexes.

In einem siebten Aspekt wird das Problem gelöst durch ein Verfahren umfassend den Schritt Nachweisen eines Antikörpers, der gegen das erfindungsgemäße Polypeptid bindet, in einer Probe eines Säugetiers, bevorzugt eines Menschen, die Antikörper enthält, bevorzugt der IgE-Klasse.

In einer ersten bevorzugten Ausführungsform des siebten Aspekts weist das Säugetier wenigstens ein Symptom einer Allergie auf und/oder es besteht der Verdacht, dass das Säugetier unter einer Allergie leidet, bevorzugt gegen Nüsse.

In einer zweiten bevorzugten Ausführungsform des siebten Aspekts, die auch eine Ausführungsform des ersten Aspektes ist, umfasst das Verfahren die Schritte
a) Bereitstellen einer Probe eines Säugetiers,
b) Kontaktieren der Probe mit dem erfindungsgemäßen Polypeptid oder der medizinischen Vorrichtung unter Bedingungen, die mit der Bildung eines Komplexes zwischen dem Polypeptid und einem Antikörper kompatibel sind, und
c) Nachweisen eines in Schritt b) gebildeten Komplexes.

Die vorliegende Erfindung basiert auf der überraschenden Erkenntnis der Erfinder, dass die Nachteile der im Stand der Technik beschriebenen Reagenzien und Verfahren durch Verwendung des erfindungsgemäßen Polypeptides überwunden werden können.

Ohne an irgendeine Theorie gebunden sein zu wollen, vermuten die Erfinder, dass an die hydrophobe Domäne der Ölkörper-assoziierten Proteine weitere Polypeptide binden, die als Antigene für diagnostische Tests eingesetzte Ölkörper-assoziierten Proteine verunreinigen. Nicht nur allergiespezifische Antikörper im Blut von Patienten, sondern auch weitere Antikörper binden an diese Verunreinigungen, was die Spezifität beeinträchtigt, beispielsweise in Form falsch-positiver Ergebnisse.

Diese Verunreinigungen können nicht an die erfindungsgemäßen rekombinanten Ölkörperassoziierten Proteine mit verkürzter hydrophober Domäne binden, da aufgrund der Verkürzung die Bindestellen fehlen. Werden diagnostische Tests auf Basis solcher rekombinanten Polypeptide aufgebaut, so ist die Gefahr falsch-positiver Ergebnisse daher verringert.

Ebenfalls ohne an irgendeine Theorie gebunden sein zu wollen, vermuten die Erfinder, dass native Ölkörper-assoziierte Proteine bei der Isolierung eine Konformation oder physikochemische Reaktionen eingehen, die zur Verdeckung von allergierelevanten Epitopen führt, was die Sensitivität beeinträchtigen kann, beispielsweise dadurch, dass allergiespezifische Antikörper in Proben nicht an das Antigen binden können.

Ebenfalls ohne an irgendeine Theorie gebunden sein zu wollen, vermuten die Erfinder weiterhin, dass die Verkürzung eine physikalisch-chemische Stabilisierung des rekombinanten Antigens im Vergleich zum nativen Volllängen-Protein bewirkt.

Die vorliegende Erfindung betrifft rekombinante, von Ölkörper-assoziierten Proteinen abgeleitete Polypeptide, ausgewählt aus der Gruppe umfassend Oleosine, Caleosine und Steroleosine, mit verkürzter hydrophober Domäne.

Oleosine, Caleosine und Steroleosine haben gemein, dass sie zu den Ölkörper-assoziierten Proteinen in Pflanzen gezählt werden. Die nativen Volllängen-Proteine dieser sind in eine hydrophobe und eine C-terminal daran angeschlossene hydrophile Domäne gegliedert. Während die Steroleosine keine weiteren Domänen aufweisen, kommt bei Oleosinen und Caleosinen noch eine N-terminale hydrophile Domäne hinzu.

Unter dem Begriff "Oleosin" wird, wie hierin verwendet, ein Polypeptid verstanden, das die Sequenz SEQ ID NO 5, bevorzugt SEQ ID NO 6 aufweist, noch bevorzugter in Kombination mit einer Sequenz aus der Gruppe umfassend SEQ ID NO 7 und SEQ ID NO 8, sowie jeweils Varianten der Sequenzen. In einer bevorzugten Ausführungsform handelt es sich um ein Oleosin aus der Gruppe umfassend diejenigen, deren Sequenzen durch die Datenbankcodes Q647G5 (Ara h 10), Q45W87 (Ara h 11), 75148112 (Q84T21, Cor a 12), 75298094 (Q84T91, Cor a 13), G8H6H8 (Jug r Oleosin-1), G8H6H9 (Jug r Oleosin-2), Q9FUJ9 (Ses i 4), Q9XHP2 (Ses i 5) und 004925 (Ses i Oleosin) dargestellt sind, besonders bevorzugt Q647G5, sowie jeweils Varianten der Sequenzen. Sämtliche Datenbankencodes entsprechen denjenigen Sequenzen, wie sie aus der jeweiligen Datenbank am 30. April 2015 abgerufen werden konnten.

Die hydrophobe Domäne ist bei einem Oleosin dadurch gekennzeichnet, dass sie die SEQ ID NO 9 und Varianten davon sowie die N-terminal davor liegenden 32, bevorzugter 29, bevorzugter 27, bevorzugter, 24 bevorzugter 20, bevorzugter 18, bevorzugter 12, bevorzugter 0, sowie die C-terminal dahinter liegenden 28, bevorzugter, bevorzugter 25, bevorzugter 15, bevorzugter 10, bevorzugter 0 Aminosäuren umfasst. Bevorzugter ist die hydrophobe Domäne bei einem Oleosin dadurch gekennzeichnet, dass sie die SEQ ID NO 10 und Varianten davon sowie die N-terminal davor liegenden 32, bevorzugter 29, bevorzugter 27, bevorzugter 24, bevorzugter 20, bevorzugter 18, bevorzugter 12, bevorzugter 0, sowie die C-terminal dahinter liegenden 28, bevorzugter, bevorzugter 25, bevorzugter 15, bevorzugter 10, bevorzugter 0 Aminosäuren umfasst.

Unter dem Begriff "Caleosin" wird, wie hierin verwendet, ein Polypeptid verstanden, das die Sequenz SEQ ID NO 11, bevorzugt SEQ ID NO 12 aufweist, sowie jeweils Varianten der Sequenzen. In einer bevorzugten Ausführungsform handelt es sich um ein Caleosin aus der Gruppe umfassend diejenigen, deren Sequenzen durch die Datenbankcodes Q9SQ57-1(peroxy Ses), 081270-1 (peroxy ara t (Arapidopsis thaliana)), Q9FLN9-1 (PXG2 Ara t), 023959-1 (Gly m Cal1), M5WJM8-1 (Pru p Cal1) dargestellt sind, besonders bevorzugt Q9SQ57-1, sowie jeweils Varianten der Sequenzen.

Die hydrophobe Domäne ist bei einem Caleosin dadurch gekennzeichnet, dass sie die SEQ ID NO 13 und Varianten davon sowie die N-terminal davor liegenden 23, bevorzugter 20, bevorzugter 10, bevorzugter 0, sowie die C-terminal dahinter liegenden 7, bevorzugter 5, bevorzugter 3, bevorzugter 2, bevorzugter 0 Aminosäuren umfasst. Bevorzugter ist die hydrophobe Domäne ist bei einem Caleosin dadurch gekennzeichnet, dass sie die SEQ ID NO 14 und Varianten davon sowie die N-terminal davor liegenden 23, bevorzugter 20, bevorzugter 10, bevorzugter 0, sowie die C-terminal dahinter liegenden 7, bevorzugter 5, bevorzugter 3, bevorzugter 2, bevorzugter 0 Aminosäuren umfasst.

Unter dem Begriff "Steroleosin" wird, wie hierin verwendet, ein Polypeptid verstanden, das die Sequenz SEQ ID NO 15, bevorzugt SEQ ID NO 16 aufweist, noch bevorzugter in Kombination mit einer Sequenz aus der Gruppe umfassend SEQ ID NOs 18, 19, 20 und 21, sowie jeweils Varianten der Sequenzen. In einer bevorzugten Ausführungsform handelt es sich um ein Steroleosin aus der Gruppe umfassend diejenigen, deren Sequenzen durch die Datenbankcodes A7LB59 (Ara h Ster), Q93W57 (Ses i Ster 1), C3S7G7 (Bra n Ster 1), C3S7G9 (Bra n Ster 2), Q8LKV5 (Ses i Ster 2), A7LB60 (Ara h Ster 2), C3S7H0 (Bra n Ster 3) und C3S7H1 (Bra n Ster 4) dargestellt sind, besonders bevorzugt um A7LB59, sowie jeweils Varianten der Sequenzen.

Die hydrophobe Domäne ist bei einem Steroleosin dadurch gekennzeichnet, dass sie die SEQ ID NO 17 und Varianten davon sowie alle N-terminal davor liegenden Aminosäuren, sowie die C-terminal dahinter liegenden 7, bevorzugter 5, bevorzugter 3, bevorzugter 2, bevorzugter 0 Aminosäuren umfasst.

In einer bevorzugten Ausführungsform wird unter dem Begriff "hydrophile Domäne" jegliche Domäne in einem Oleosin, Caleosin oder Steroleosin außerhalb der hydrophoben Domäne verstanden. Im Falle einer N-terminalen hydrophilen Domäne handelt es sich um die in der Primärsequenz vor der hydrophoben Domäne liegende Domäne. Im Falle einer C-terminalen hydrophilen Domäne handelt es sich um die in der Primärsequenz hinter der hydrophoben Domäne liegende Domäne.

Das erfindungsgemäße Polypeptid umfasst ein antigenes Fragment mit einer Sequenz von wenigstens sieben Aminosäuren aus einer hydrophilen Domäne eines Ölkörper-assoziierten Proteins, wobei das Polypeptid keine oder eine verkürzte hydrophobe Domäne des Ölkörperassoziierten Proteins aufweist, was in einer bevorzugten Ausführungsform die Abwesenheit der vollständigen hydrophoben Domäne aus dem entsprechenden Wildtyp-Protein bedeutet. Die verkürzte hydrophobe Domäne weist in der Reihenfolge zunehmender Bevorzugung eine Länge von 99, 95, 90, 80, 70, 60, 50, 40, 30, 20, 10 oder 5 % ihrer Länge im entsprechenden Wildtyp-Polypeptid auf. Dabei ist die hydrophobe Domäne bevorzugt in der Weise verkürzt, dass es nicht zur Ausbildung einer Transmembrandomäne kommt. In einer bevorzugten Ausführungsform ist unter dem Begriff "antigenes Fragment" aus einem Polypeptid eine daraus entnommene Aminosäuresequenz mit wenigstens 7, 10, 15, 20, 30 oder 40 aufeinanderfolgenden Aminosäureresten zu verstehen, das spezifisch gegen Antikörper der Klasse IgE aus Patienten mit entsprechender Allergie bindet.

In einer bevorzugten Ausführungsform umfasst das erfindungsgemäße Polypeptid zusätzlich ein die Löslichkeit in wässriger Lösung erhöhendes Polypeptid. Dem Fachmann sind zahlreiche geeignete Fusionsproteine bekannt, beispielsweise MPB, GST, Protein G B1-Domain, Thioredoxin, NusA, Ubiquitin, SUMO und T7gene10. Dieses die Löslichkeit erhöhende Polypeptid kann N-terminal, C-terminal zu oder zwischen den antigenen Fragmenten angeordnet sein, bevorzugt liegt es N-terminal.

In einer bevorzugten Ausführungsform wird unter dem Begriff "Linker", wie hierin verwendet, eine flexible Kette von Aminosäuren verstanden, bevorzugt einer Länge von 1 bis 100, 2 bis 80, 3 bis 50 oder 4 bis 40 Aminosäuren. Der Linker dient dazu, Fragmente kovalent zu verbinden, ohne ihre sterische Zugänglichkeit einzuschränken. Eine mögliche Linkersequenz ist in SEQ ID NO 22 dargestellt, ein beispielhaftes Gesamtkonstrukt damit in SEQ ID NO 23.

Die Lehre der vorliegenden Erfindung kann nicht nur unter Verwendung von Makromolekülen mit der exakten Aminosäure- oder Nukleinsäuresequenz, auf die hierin Bezug genommen wird, ausgeführt werden, beispielsweise antigene Fragmente von Ölkörper-assoziierten Proteinen, sondern auch unter Verwendung einer *Variante* derartiger Makromoleküle, die durch Deletion, Addition oder Substitution einer oder mehr als einer Aminosäure oder Nukleinsäure erhalten werden kann. In einer bevorzugten Ausführungsform bedeutet der Begriff "Variante" einer Nukleinsäuresequenz oder Aminosäuresequenz, wie hierin verwendet, eine andere Sequenz, welche mit Hinblick auf die erstgenannte Nukleinsäuresequenz oder Aminosäuresequenz eine Homologie von in der Reihenfolge zunehmender Bevorzugung 70, 75, 80, 85, 90, 92, 94, 96, 98, 99 % oder mehr Prozent aufweist, wobei bevorzugt andere als die für die biologische Aktivität wichtige Aminosäuren in einer entsprechenden exprimierten Aminosäuresequenz verändert sind. Dabei bedeutet der Begriff "Homologie" entweder Identität von Aminosäuren oder konservativen Austausch gegen eine andere Aminosäure, bevorzugt Identität. In einer bevorzugteren Ausführungsform bedeutet ein konservativer Austausch dabei, dass eine Aminosäure aus den folgenden Gruppen gegen eine andere Aminosäure der jeweiligen Gruppe ausgetauscht ist: hydrophobe Aminosäuren (V, L, I), hydroxylgruppenhaltige Aminosäuren (T, S), saure Aminosäuren (D, E), amidische Aminosäuren (N, Q), basische Aminosäuren (K, R) und aromatische Aminosäuren (F, W).

In einer bevorzugteren Ausführungsform der vorliegenden Erfindung weist die Variante einer Aminosäure- oder Nukleinsäuresequenz, bevorzugt zusätzlich zur oben genannten Sequenzhomologie, im Wesentlichen die gleiche biologische Aktivität des Wildtypmoleküls bzw. des ursprünglichen Moleküls auf. Zum Beispiel weist eine Variante eines spezifisch gegen einen bestimmten Antikörper bindenden Polypeptids die gleiche oder im Wesentlichen die gleiche Bindungsspezifität auf wie das Wildtypmolekül. In einer besonderen Ausführungsform bedeutet der Begriff "im Wesentlichen die gleiche Bindungsspezifität" eine Dissoziationskonstante, die sich um nicht weniger als einen Faktor 1000, bevorzugter 500, noch bevorzugter 100, noch bevorzugter 50, noch bevorzugter 20, noch bevorzugter 10, noch bevorzugter 2 von der Dissoziationskonstante bei der Bindungsreaktion zwischen dem gleichen Antikörper mit der erstgenannten, ursprünglichen Sequenz unterscheidet.

Die Variante der Nukleinsäure- oder Aminosäuresequenz weist die gleiche Länge der erstgenannten, ursprünglichen Sequenz oder ein Fragment davon auf, beispielsweise ein Fragment mit wenigstens 7, bevorzugter 10, 20, 30, 40, 50, 60, 70, 80, 90, 100, 120, 150 oder 200 Aminosäuren auf, wobei das Fragment am C-Terminus, N-Terminus oder dazwischen, bevorzugt N-Terminus, einer N-terminal von der hydrophoben Domäne liegenden hydrophilen Domäne beginnen kann oder das Fragment am N-Terminus einer C-terminal von der hydrophoben Domäne liegenden hydrophilen Domäne liegen kann. Bevorzugt umfasst das Fragment in der Reihenfolge zunehmender Bevorzugung 20, 30, 40, 50, 60, 70, 80, 90, 95, 99 oder 100 % der Aminosäuren der jeweiligen N-terminalen oder C-terminalen Domäne.

Es ist essentiell, dass die Variante des Polypeptides biologische Aktivität aufweist, d.h. spezifisch an nussallergiespezifische Antikörper in Proben von Patienten mit Nussallergie zu binden, bevorzugt an Antikörper der Klasse IgE. Mit Seren von Patienten, die unter Nussallergien leiden, und üblichen Nachweismethoden, bevorzugt mit einem Westernblot, kann überprüft werden, ob diese biologische Aktivität vorliegt.

Das erfindungsgemäße Polypeptid kann zur Ausführung der vorliegenden Erfindung jeglicher Form und in jeglichem Reinheitsgrad bereitgestellt werden, von Geweben oder Zellen, die das Polypeptid exprimieren, bevorzugt Zellen, die das Polypeptid überexprimieren, kruden oder angereicherten Lysaten derartiger Zellen, bis zu aufgereinigtem und/oder isoliertem Polypeptid, das im Wesentlichen rein ist. Das erfindungsgemäße Polypeptid kann ein ungefaltetes, chemisch synthetisiertes Peptid sein. Das erfindungsgemäße Polypeptid kann ein gefaltetes Polypeptid sein. In einer bevorzugten Ausführungsform ist das Polypeptid ein rekombinantes Polypeptid, wobei der Begriff "rekombinant", wie hierin verwendet, ein Polypeptid bezeichnet, das unter Verwendung von gentechnischen Methoden an irgendeiner Stufe des Produktionsprozesses hergestellt wurde, z. B. durch das Fusionieren einer Nucleinsäure, die das Polypeptid kodiert, an einen stärkeren Promotor zur Überexpression in Zellen oder Geweben oder durch Verändern der Sequenz des Polypeptides oder einer dafür kodierenden Nukleinsäure selbst. Der Fachmann auf dem Gebiet ist mit Verfahren mit gentechnischen Methoden zum Verändern von Nukleinsäuren und Polypeptiden vertraut (siehe z. B. Sambrook, J., Fritsch, E. F. and Maniatis, T. (1989), Molecular Cloning, CSH oder Brown T. A. (1986), Gene Cloning - an introduction, Chapman & Hall) und ist in der Lage rekombinante Polypeptide herzustellen und aufzureinigen (siehe z. B. Handbücher "Strategies for Protein Purification", "Antibody Purification", "Purifying Challenging Proteins", "Recombinant Protein Purification", "Affinity Chromatography", "Ion Exchange Chromatography", "Gel Filtration (Size Exclusion Chromatography)", "Hydrophobic Interaction Chromatography", "Multimodal Chromatography" (2009/2010), veröffentlicht von GE Healthcare Life Sciences, und in Burgess, R. R., Deutscher, M. P. (2009), Guide to Protein Purification,). In einer bevorzugten Ausführungsform ist ein Polypeptid rein, wenn wenigstens 60, 70, 80, 90, 95 oder 99 Prozent des gesamtes Polypeptides in der jeweiligen Probe aus dem Polypeptid besteht, wie durch visuelle Beurteilung nach SDS PAGE gefolgt von Coomassie-Blaufärbung beurteilt werden kann.

Sofern das Polypeptid in Form einer rekombinanten Zelle bereitgestellt wird, kann es sich bei der Zelle um eine eukaryotische Zelle, wie eine Hefezelle, oder eine prokaryotische Zelle, wie *Escherichia coli,* handeln. Z. B. kann es sich bei der Zelle um eine HEK293-Zelle handeln, die mit dem der Nukleinsäure transfiziert ist, welche für das erfindungsgemäße Polypeptid funktionell kodiert. Der Fachmann auf dem Gebiet ist mit Verfahren zum Herstellen, Transfizieren und Kultivieren derartiger Zellen vertraut, wie beispielsweise in Phelan, M. C. (2001), Basic Techniques in Mammalian Cell Tissue Culture, John Wiley beschrieben ist.

Das Polypeptid, das zur Ausführung der erfindungsgemäßen Lehre eingesetzt wird, sowie jegliche Varianten davon, sind derart gestaltet, dass es Epitope aufweist, die von Antikörpern in Seren von Patienten erkannt werden, die an einer Krankheit leiden, bevorzugt an einer Allergie, bevorzugter eine Allergie gegen Pflanzenteile, bevorzugter gegen Samen und Kerne, noch bevorzugter gegen Nüsse.

Ein solches Polypeptid weist einen Abschnitt von wenigstens 7, 8, 9, 10, 11, 12, 20, 25, 30, 40, 50, 60, 70, 80, 90, 100 oder mehr aufeinander-folgende Aminosäuren aus einem Ölkörperassoziierten Protein auf. Der Fachmann auf dem Gebiet ist mit Richtlinien vertraut, die herangezogen werden können, um Peptide ausreichender Immunogenität zu gestalten, wie sie beispielsweise in Jackson, D. C., Fitzmaurice, C. J., Brown, L. E., Zeng, W. (1999), Preparation and properties of totally synthetic immunogenes, Vaccine Volume 18, Issues 3-4, September 1999, Seiten 355-361; und Black, M., Trent, A., Tirrell, M. and Olive, C. (2010), Advances in the design and delivery of peptide subunit vaccines with a focus on Toll-like receptor agonists, Expert Rev Vaccines, 2010 Februar; 9(2): 157-173) beschrieben sind. Kurz gesagt sollte das Peptid so viele der folgenden Eigenschaften aufweisen wie möglich: (a) es ist stark hydrophil, (b) es umfasst wenigstens einen Rest auf der Gruppe Aspartat, Prolin, Tyrosin und Phenylalanin, (c) es hat zum Erreichen einer hohen Spezifität keine oder nur eine geringe Homologie mit anderen bekannten Peptiden oder Polypeptiden, (d) es muss ausreichend löslich sein und (e) es umfasst keine Glykolisierungs- oder Phosphorylierungsstellen, es sei denn, dass diese aus spezifischen Gründen erforderlich sind. Alternativ können bioinformatische Ansätze verwendet werden, beispielsweise diejenigen, die in Moreau, V., Fleury, C., Piquer, D., Nguyen, C., Novali, N., Villard, S., Laune, D., Granier, C. and Molina, F. (2008), PEPOP: Computational design of immunogenic peptides, BMC Bioinformatics 2008, 9:71 beschrieben sind.

Das erfindungsgemäße Polypeptid kann in jeglicher Konformation bereitgestellt werden. Z. B. kann das Polypeptid im Wesentlichen ungefaltet, teilweise oder vollständig gefaltet sein. In einer bevorzugten Ausführungsform ist das Polypeptid in dem Sinne gefaltet, dass die zum Binden des Antikörpers erforderlichen Epitope die Faltung annehmen, die das native Protein in seiner natürlichen Umgebung annimmt. Der Fachmann auf dem Gebiet ist mit den Verfahren vertraut, mittels derer bestimmt werden kann, ob ein Polypeptid gefaltet ist, und wenn ja, welche Struktur es annimmt, z. B. emittierte Proteolyse, NMR-Spektroskopie, CD-Spektroskopie oder Röntgenkristallographie (siehe z. B. Banaszak L. J. (2008), Foundations of Structural Biology, Academics Press, or Teng Q. (2013), Structural Biology: Practical Applications, Springer).

Zur Aufreinigung oder Immobilisierung kann das erfindungsgemäße Polypeptid ein Tag aufweisen, eine Sequenz oder Domäne, die in der Lage ist, spezifisch an einen Liganden zu binden, beispielsweise ein Tag aus der Gruppe umfassend CBD, CBP, FLAG, c-Myc, Strep-Tag, Poly-Arg, His-Tag, Thioredoxin, MBP und GST.

Das erfindungsgemäße Polypeptid kann ein immobilisiertes Polypeptid sein. In einer bevorzugten Ausführungsform bedeutet der Begriff "immobilisiert", wie hierin verwendet, ein Molekül, das an einen festen Träger gebunden ist, welcher in wässriger Lösung unlöslich ist, bevorzugter über eine kovalente Bindung, elektrostatische Interaktionen, Einkapselung oder Einfangen, z. B. durch Denaturieren eines globaleren Polypeptides in einem Gel, bevorzugter über hydrophobe Interaktion, am bevorzugtesten über eine oder mehr als eine kovalente Bindungen. Verschiedene geeignete Träger, z. B. Papier, Polystyrol, Metall, Silikon oder Glasoberflächen, Mikrofluidkanäle, Membran, Mikrokügelchen, wie magnetische Mikrokügelchen, Säulenchromatographie-Medien, Biochips, Polyacrylamidgele und dergleichen sind in der Literatur beschrieben, z. B. in Kim et al. Protein immobilizsation techniques for microfluidic assays, Biomicrofluidics 7(4), 041501. Auf diese Weise kann das immobilisierte Molekül zusammen mit dem unlöslichen Träger von einer wässrigen Lösung auf einfache Weise getrennt werden, z. B. durch Filtration, Zentrifugation oder Dekantieren. Das immobilisierte Molekül kann reversibel oder irreversible immobilisiert sein. Z. B. ist die Immobilisierung reversibel wenn das Molekül mit dem Träger über ionische Interaktionen interagiert, die durch Zugabe einer hohen Konzentration an Salz maskiert werden kann oder wenn das Molekül über eine lösbare kovalente Bindung, wie beispielsweise eine Disulfid-Brücke gebunden ist, die durch Zugabe von thiolhaltigen Reagenzien gespalten werden kann. Das Polypeptid kann indirekt immobilisiert sein, z. B. durch Immobilisieren eines Antikörpers oder eines anderen Moleküls, das eine Affinität zu dem Polypeptid hat, gefolgt von Bildung eines Komplexes mit der Wirkung, dass der Polypeptid-Antikörperkomplex immobilisiert ist. Verschiedene Verfahren zum Immobilisieren von Polypeptiden sind in der Literatur beschrieben, z. B. in Kim et al. Verschiedene Reagenzien und Kits für Immobilisierungsreaktionen sind im Handel erhältlich, z. B. von Pierce Biotechnology.

Erfindungsgemäß wird ein Kit bereitgestellt, bevorzugt zum Diagnostizieren einer Krankheit. Ein solcher Kit kann Anweisungen zur Benutzung des Kits und ein Mittel zum Kontaktieren des erfindungsgemäßen Polypeptides mit einer flüssigen Körperprobe von einem Subjekt enthalten, bevorzugt einem menschlichen Subjekt, zum Beispiel einen Linienblot, wobei das erfindungsgemäße Polypeptid auf dem Linienblot immobilisiert ist. Die Durchführung von linienblot-basierten Tests und ihr Aufbau ist im Stand der Technik beschrieben ((Raoult, D., and Dasch, G. A. (1989), The line blot: an immunoassay for monoclonal and other antibodies. Its application to the serotyping of gram-negative bacteria. J. Immunol. Methods, 125 (1- 2), 57-65; WO2013041540).

Weiterhin kann der Kit eine Positivkontrolle umfassen, beispielsweise einen Antikörper, von dem bekannt ist, dass er an das erfindungsgemäße Polypeptid bindet, und eine Negativkontrolle, zum Beispiel ein Protein, das keine nachweisbare Affinität zum erfindungsgemäßen Polypeptid aufweist, beispielsweise Rinderserumalbumin. Schließlich kann ein solcher Kit eine Standardlösung des Antikörpers oder Polypeptides zur Kalibrierung umfassen.

Gemäß der Erfindung wird ein Antikörper bereitgestellt, der in Lage ist, spezifisch an das erfindungsgemäße Polypeptid zu binden. In einer bevorzugten Ausführungsform bedeutet der Begriff "Antikörper", wie hierin verwendet, jegliche Bindungseinheiten auf Immunglobulinbasis, bevorzugter wenigstens eine Immunglobulin-schwere Kette und eine Immunglobulin-leichte Kette, umfassend aber nicht beschränkt auf monoklonale und polyklonale Antikörper wie auch Varianten eines Antikörpers, insbesondere Fragmente, die in der Lage sind, an das Antigen zu binden, bevorzugt sehr spezifisch zu binden. In einer besonderen Ausführungsform bedeutet der Begriff "spezifisch Binden", wie hierin verwendet, dass die Bindung stärker als eine Bindungsreaktion ist, die durch eine Dissoziationskonstante, vor allem einer Dissoziationskonstante von 1 x 10⁻⁵ M, bevorzugter 1 x 10⁻⁷ M, bevorzugter 1 x 10⁻⁸ M, bevorzugter 1 x 10⁻⁹ M, bevorzugter 1 x 10⁻¹⁰ M, bevorzugter 1 x 10⁻¹¹ M, bevorzugter 1 x 10⁻¹² M gekennzeichnet ist, wie durch Surface Plasmon Resonance unter Verwendung von Biacore-Geräten bei 25 °C in PBS-Puffer pH 7 bestimmt wird. Der Antikörper kann isoliert und in einer Mischung umfassend weitere Antikörper, Polypeptide, Metabolite, Zellen und dergleichen vorliegen.

Die Reagenzien, Vorrichtungen, Verfahren und Verwendungen, die in dieser Anmeldung beschrieben werden, können zur Diagnose einer Krankheit verwendet werden. In einer bevorzugten Ausführung handelt es sich bei dieser Krankheit um eine Allergie. In einer noch bevorzugten Ausführungsform handelt es sich um eine Allergie gegen Nüsse, bevorzugt gegen wenigstens eine Nuss aus der Gruppe umfassend Haselnüsse, Sesam, Erdnüsse, Walnüsse und Pekannüsse. In einer bevorzugten Ausführungsform bedeutet der Begriff "Diagnose", wie hierin verwendet, jegliche Art von Vorgehen, das darauf abzielt, Informationen zu erhalten, die die Beurteilung unterstützen, ob ein Subjekt von einer bestimmten Krankheit in der Vergangenheit zur Zeit der Diagnose oder in der Zukunft litt, leidet oder leiden wird, oder dass dies wahrscheinlicher ist als bei einem durchschnittlichen Vergleichssubjekt, welches ähnliche klinische Symptome aufweist, oder um herauszufinden, wie die Krankheit fortschritt oder in der Zukunft wahrscheinlich fortschreiten wird, oder um das Ansprechen des Patienten auf eine bestimmte Art von Behandlung zu evaluieren. Mit anderen Worten umfasst der Begriff "Diagnose" nicht nur das Diagnostizieren, sondern auch das Prognostizieren oder das Verfolgen des Verlaufs einer Krankheit.

In vielen Fällen ist das bloße Nachweisen des Antikörpers ausreichend, mit anderen Worten, dass die Bestimmung, ob nachweisbare Konzentrationen des Antikörpers in der Probe vorliegen. Wenn der Antikörper nachgewiesen wird, ist dies eine wesentliche Information für die Diagnose des betreuenden Arztes und zeigt eine erhöhte Wahrscheinlichkeit an, dass der Patient an der Krankheit leidet. In einer bevorzugten Ausführungsform kann die relative Konzentration des Antikörpers im Serum mit der Konzentration verglichen werden, die in einem durchschnittlichen gesunden Subjekt gefunden wird. Zum erfindungsgemäßen Verfahren kann gehören, dass bestimmt wird, ob die Konzentration des Antikörpers wenigstens 0,1, bevorzugt 0,2, 0,5, 1, 2, 5, 10, 20, 25, 50, 100, 200, 500, 1.000, 10.000 oder 100.000 mal höher ist als diejenige Konzentration, die in einem durchschnittlichen gesunden Subjekt gefunden würde.

Der Fachmann auf dem Gebiet erkennt an, dass der praktizierende behandelnde Arzt seine Diagnose nicht ausschließlich auf der Grundlage eines einzelnen diagnostischen Parameters stellt, sondern zahlreiche Aspekte in Betracht ziehen muss, z. B. die Anwesenheit anderer Antikörper, Marker, Blutparameter, klinische Untersuchung der Symptome des Patienten oder die Ergebnisse von Bildgebung oder anderen nichtinvasiven Verfahren, um die Gesamtdiagnose zu erstellen. Der Wert eines diagnostischen Agens oder eines diagnostischen Verfahrens kann auch darin bestehen, eine oder mehrere Krankheiten auszuschließen und dadurch die indirekte Diagnose einer anderen Krankheit zu gestatten. In einer bevorzugten Ausführungsform ist die Bedeutung jeglicher Symptome oder Krankheiten, auf die in dieser Anmeldung Bezug genommen wird, im Einklang mit dem Verständnis des Fachmanns am Anmeldetag zu sehen, wie es an diesem Tag durch Lehrbücher und wissenschaftliche Publikationen dargestellt wird.

Demnach bedeutet der Begriff "Diagnose" bevorzugt nicht, dass die erfindungsgemäßen diagnostischen Verfahren oder Reagenzien ein definitives und ausreichendes Ergebnis ergeben werden. Um die Diagnose auf der Grundlage eines einzelnen Tests oder gar Parameters endgültig zu stellen, kann ein Beitrag zu einer "differentiellen Diagnose" bedeuten, d. h. einer systematischen diagnostischen Vorgehensweise, die die Wahrscheinlichkeit von einer Reihe von Krankheiten auf der Basis von einer Reihe von diagnostischen Parametern betrachtet. Der Begriff "Diagnose" kann auch bedeuten, dass ein Verfahren oder Reagenz benutzt wird, um zwischen zwei oder mehr Krankheiten mit ähnlichen Symptomen zu unterscheiden.

Der Begriff "Diagnose" kann auch bedeuten, dass bestimmt wird, welches Reagenz oder Behandlungsverfahren unter mehreren zur Verfügung stehenden Optionen für den Patienten am verheißungsvollsten ist.

Das erfindungsgemäße Polypeptid kann in einer Kombination mit weiteren allergiespezifischen Antigenen verwandt werden. Dabei kann eine Mischung oder eine Kombination mit mehreren voneinander getrennt vorliegenden aufgereinigten Antigen eingesetzt werden, bevorzugt in immobilisierter Form, noch bevorzugter auf einer medizinischen Vorrichtung wie einem Linienblot. Durch Kombination verschiedener isolierter Allergene kann für jeden Patienten ein individuelles Sensibilisierungsmuster erstellt werden und damit Hinweise auf den Schweregrad der bestehenden Allergie oder auch über den zukünftigen Krankheitsverlauf gewonnen werden.

Ein erfindungsgemäßes Polypeptid umfassend ein antigenes Fragment mit einer Sequenz von wenigstens sieben Aminosäuren aus einer hydrophilen Domäne aus einem Erdnuss-Oleosin, - Caleosin oder -Steroleosin umfasst, bevorzugt aus einem Erdnuss-Oleosin, kann kombiniert werden mit einem oder mehr als einem Allergen oder einer Variante davon aus der Gruppe umfassend Ara h 1 (P43238), Ara h 2 (Q6PSU2), Ara h 6 (Q647G9), Ara h 9 (B6CG41), kombiniert werden, bevorzugter aus der Gruppe umfassend Ara h 1, Ara h 2, Ara h 6, noch bevorzugter Ara h 2 und Ara h 6, am bevorzugtesten mit Ara h 2.

Ein erfindungsgemäßes Polypeptid umfassend ein antigenes Fragment mit einer Sequenz von wenigstens sieben Aminosäuren aus einer hydrophilen Domäne aus einem Haselnuss-Oleosin, -Caleosin oder -Steroleosin umfasst, bevorzugt aus einem Haselnuss-Oleosin, kann kombiniert werden mit einem oder mehr als einem Allergen oder einer Variante davon aus der Gruppe umfassend Cor a 1 (Q9SWR4), Cor a 2 (Q9AXH5), Cor a 8 (Q9ATH2), Cor a 9 (Q8W1 C2) und Cor a 14 (D0PWG2), bevorzugter aus der Gruppe umfassend Cor a 1, Cor a 8, Cor a 9 und Cor a 14, bevorzugter Cor a 1, Cor a 9, und Cor a 14, noch bevorzugter Cor a 9, und Cor a 14, am bevorzugtesten mit Cor a 14.

Ein erfindungsgemäßes Polypeptid umfassend ein antigenes Fragment mit einer Sequenz von wenigstens sieben Aminosäuren aus einer hydrophilen Domäne aus einem Sesam-Oleosin, - Caleosin oder -Steroleosin umfasst, bevorzugt aus einem Sesam-Oleosin, kann kombiniert werden mit einem oder mehr als einem Allergen oder einer Variante davon aus der Gruppe umfassend Ses i 1 (Q9AUD1), Ses i 2 (Q9XHP1), Ses i 3 (Q9AUD0), Ses i 6 (Q9XHP0) und Ses i 7 (Q9AUD2), bevorzugter aus der Gruppe umfassend Ses i 1, Ses i 2, Ses i 3, und Ses i 6, noch bevorzugter Ses i 1, Ses i 2, und Ses i 3, noch bevorzugter Ses i 1, Ses i 2, am bevorzugtesten mit Ses i 2.

In einer bevorzugten Ausführungsform wird der Nachweis des Komplexes mit einem Verfahren aus der Gruppe ausgeführt, die Immundiffusionstechniken, immunelektrophoretische Techniken, Lichtstreuungs-Immunassays, Agglutinationstechniken, Markierungen, wie aus der Gruppe Radio-Immunassay, Enzym-Immunassay, Chemilumineszenz-Immunassay und Immunfluoreszenztechniken ausgeführt werden. Der Fachmann auf dem Gebiet ist mit diesem Verfahren vertraut, die auch im Stand der Technik beschrieben sind, z. B. in Zane, H. D. (2001), Immunology - Theoretical & Practical Concepts in Laboratory Medicine, W. B. Saunders Company, besonders in Kapitel 14.

Der Schritt "Kontaktieren einer flüssigen Probe umfassend Antikörper mit dem erfindungsgemäßen Polypeptid" wird unter geeigneten Bedingungen ausgeführt. In einer bevorzugten Ausführungsform bedeutet der Begriff "Bedingungen, die mit der Bildung des Komplexes kompatibel sind" Bedingungen, die es erlauben, dass die spezifischen Antigen-Antikörper-Wechselwirkungen den Komplex umfassend das Polypeptid und den Antikörper aufbauen. In einer bevorzugten Ausführung können solche Bedingungen das Inkubieren des Polypeptides in einer 1:100 verdünnten Probe in PBS Puffer 30 Minuten lang bei 25 °C umfassen. Jegliche Daten, die Anwesenheit oder Abwesenheit des Komplexes umfassend den Antikörper und das erfindungsgemäße Polypeptid umfassen, können mit Referenzdaten korreliert werden. Z. B. kann der Nachweis des Komplexes anzeigen, dass der Patient, von dem die Probe stammt, an einer Krankheit litt oder in Zukunft leiden wird. Wenn der Patient zuvor diagnostiziert wurde und das Verfahren zum Diagnostizieren noch einmal aufgeführt wird, kann die Komplexmenge, die in beiden Durchläufen nachgewiesen wurde, miteinander korreliert werden, um das Fortschreiten der Krankheit und/oder den Behandlungserfolg zu evaluieren. Wenn die Menge des Komplexes z. B. zunimmt, legt dies nahe, dass die Krankheit fortschreitet, sich wahrscheinlich in Zukunft manifestieren wird und/oder dass die Behandlung nicht erfolgreich ist.

Es ist erforderlich, dass die Probe, die für das diagnostische erfindungsgemäße Verfahren eingesetzt wird, Antikörper enthält, die auch als Immunglobuline bezeichnet werden. Typischerweise umfasst die Probe eine repräsentative Menge der Gesamtheit der Antikörper des zu untersuchenden Subjektes. Die Probe kann nach der Bereitstellung jedoch auch prozessiert werden, was eine Fraktionierung, Zentrifugation, Anreicherung oder Isolierung der Gesamtheit der Antikörper oder einer bestimmten Antikörperklasse umfassen kann, was die relative Verteilung von Immunglobulin der verschiedenen Klassen derart beeinflussen kann, dass sie von der ursprünglichen Verteilung abweicht. In einer bevorzugten Ausführungsform sind Immunglobuline der Klasse IgE angereichert.

Das therapeutische Polypeptid kann therapeutisch verwandt werden bzw. zur Herstellung eines Medikamentes, beispielsweise eines Reagenz zur Desensibilisierung oder als Impfstoff. Es liegt dabei in pharmazeutisch akzeptabler Form vor, beispielsweise in Kombination mit einem Träger.

Der erfindungsgemäße oder erfindungsgemäß nachgewiesene Antikörper ist ein Antikörper der IgE- oder IgG-Klasse, beispielsweise IgG4, besonders bevorzugt der IgE-Klasse. Der Fachmann auf dem Gebiet ist mit Methoden zum Aufreinigungen Antikörpern vertraut, beispielsweise denjenigen die in Hermanson et al. beschrieben sind.

In dieser Anmeldung und dem beigefügten Sequenzprotokoll wird auf eine Reihe von Sequenzen Bezug genommen, nämlich

| SEQ ID NO | Beschreibung |
|---|---|
| SEQ ID NO 1 | N-terminale hydrophile Domäne des Oleosins aus der Haselnuss |
| SEQ ID NO 2 | C-terminale hydrophile Domäne des Oleosins aus der Haselnuss |
| SEQ ID NO 3 | N-terminale hydrophile Domäne des Oleosins aus der Walnuss |
| SEQ ID NO 4 | C-terminale hydrophile Domäne des Oleosins aus der Walnuss |
| SEQ ID NO 5 | Konsensus-Sequenz I von Oleosinen |
| SEQ ID NO 6 | Konsensus-Sequenz II von Oleosinen |
| SEQ ID NO 7 | C-terminales Sequenzmotiv I von Oleosinen |
| SEQ ID NO 8 | C-terminales Sequenzmotiv II von Oleosinen |
| SEQ ID NO 9 | Konsensus-Sequenz I für hydrophobe Domäne von Oleosinen |
| SEQ ID NO 10 | Konsensus-Sequenz II für hydrophobe Domäne von Oleosinen |
| SEQ ID NO 11 | Konsensus-Sequenz I von Caleosinen |
| SEQ ID NO 12 | Konsensus-Sequenz II von Caleosinen |
| SEQ ID NO 13 | Konsensus-Sequenz I für hydrophobe Domäne von Caleosinen |
| SEQ ID NO 14 | Konsensus-Sequenz II für hydrophobe Domäne von Caleosinen |
| SEQ ID NO 15 | Konsensus-Sequenz I von Steroleosinen |
| SEQ ID NO 16 | Konsensus-Sequenz II von Steroleosinen |
| SEQ ID NO 17 | Konsensus-Sequenz I für hydrophobe Domäne von Steroleosinen |
| SEQ ID NO 18 | C-terminales Sequenzmotiv I von Steroleosinen |
| SEQ ID NO 19 | C-terminales Sequenzmotiv II von Steroleosinen |
| SEQ ID NO 20 | C-terminales Sequenzmotiv III von Steroleosinen |
| SEQ ID NO 21 | C-terminales Sequenzmotiv IV von Steroleosinen |
| SEQ ID NO 22 | Beispielhafte Linkersequenz |
| SEQ ID NO 23 | Beispielhaftes Konstrukt abgeleitet von Cor a |

Im Folgenden wird die Erfindung unter Bezugnahme auf die Figuren anhand von Ausführungsbeispielen erläutert. Die beschriebenen Ausführungsformen sind in jeder Hinsicht lediglich beispielhaft und nicht als einschränkend zu verstehen, und verschiedene Kombinationen der angeführten Merkmale sind vom Umfang der Erfindung umfasst.

Fig. 1 zeigt eine SDS-PAGE-Analyse des mittels IMAC gereinigten rekombinanten Cor a 12. Die Analyse erfolgte mit einem 4-12-%igen Bis-Tris-Gel und anschließender Coomassie-Färbung. Spur 1: Broad Range Marker, Spur 2: gereinigtes, rekombinantes Cor a 12 (nicht reduziert), Spur 3: gereinigtes, rekombinantes Cor a 12 (reduziert).

Fig. 2 zeigt einen mit Cor a 12 beschichteten Linienblot.

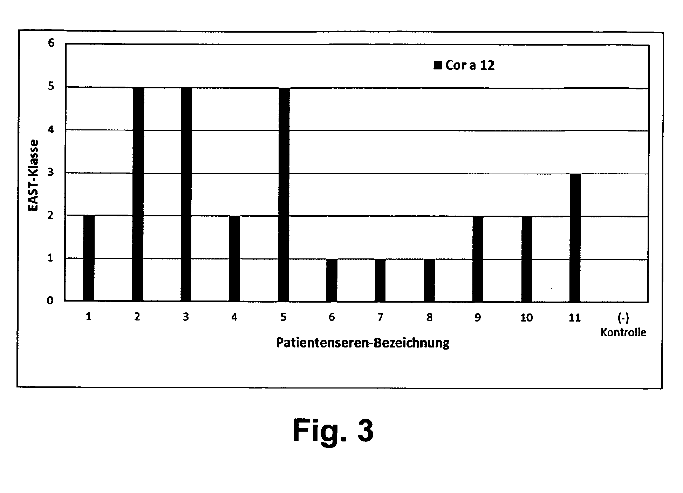

Fig. 3 zeigt eine Reaktivitätsanalyse des erfindungsgemäßen Polypeptides Cor a 12 der Haselnuss im Euroline. Die Reaktivitätsanalyse erfolgte mit Haselnuss-Allergiker-Seren. Die Auswertung der gebundenen Antikörper aus den Patientenseren erfolgte mit dem EAST-Referenzsystem.

### Beispiel 1: Klonierung, rekombinante Expression und Reinigung von Cor a 12

Die codierende Region des modifizierten Cor a 12 (SEQ ID NO 23) wurde mittels Gensynthese (Eurofins Genomics GmbH) generiert, durch Polymerase-Ketten-Reaktion amplifiziert und unter Verwendung des pBAD/TOPO® ThioFusion™ Expression Kits (Invitrogen) in den rekombinanten Vektor subkloniert. Das Fusionsprotein wurde den Herstellerangaben nach in *E. coli* exprimiert und affinitätschromatografisch über eine Nickelmatrix (IMAC) gereinigt.

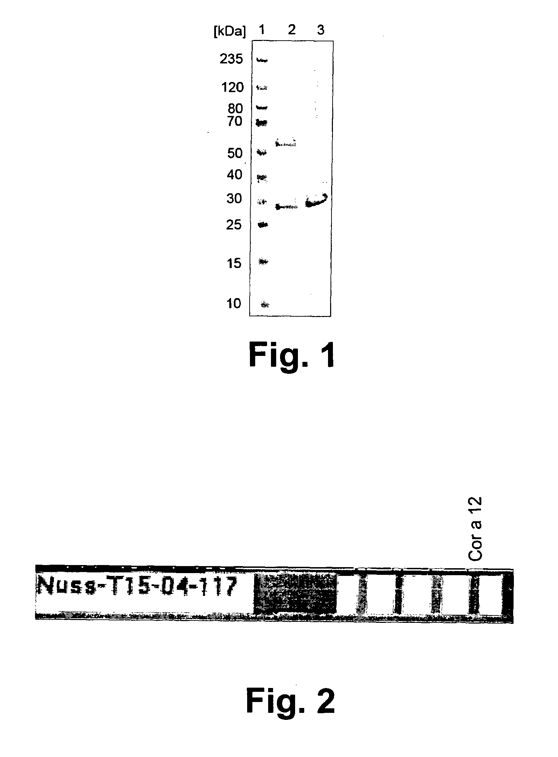

Das rekombinante Konstrukt wurde mittels SDS-PAGE analysiert, in Fig. 1 ist das mit Coomassie angefärbte SDS-Gel gezeigt. Es wurden 3 µg Protein pro Spur aufgetragen.

### Beispiel 2: Herstellung eines Linienblots (EUROLINE)

Beim Euroline wurden die erfindungsgemäßen Proteine mit Hilfe eines Präzisiondispensierers (Zeta Corporation) direkt auf eine Nitrocellulose-Membran (SchleicherSchüll) aufgetragen. Dabei werden homogene Linien gezogen. Die Membranen wurden anschließend nach Herstellerangaben blockiert und gewaschen und in 3 mm breite Streifen geschnitten.

### Beispiel 3: Durchführung des erfindungsgemäßen Tests

Jeder Streifen wurde separat mit 1 ml der 1:10 (v/v) in Waschpuffer (20 mM Tris, pH 7,5) verdünnten Patientenprobe in Kontakt gebracht, für 16 h bei Raumtemperatur inkubiert, das Serum entfernt und der Streifen anschließend 3 mal für je 5 Min mit 1 ml Waschpuffer gewaschen. Nach Abgießen des Waschpuffers wurde jeder Streifen mit je 1 ml Anti-human IgE-Antikörper AP-konjugiert (Abcam, 1:1000 (v/v) verdünnt in Waschpuffer) bei Raumtemperatur inkubiert. Nach einer Stunde wurde das Konjugat verworfen, die Streifen erneut dreimal mit 1 ml Waschpuffer gewaschen, für 10 Minuten mit BCIP/NBT-Substrat inkubiert und schließlich die Reaktion mit aqua dest. abgestoppt.

Die Auswertung erfolgte mittels Scanner und EUROLlNEScan®-Programm (EUROIMMUN) erfolgen. Eine semiquantitative Beurteilung kann anhand einer IgE-Kalibrationskurve nach WHO-Standard (75/502) durchgeführt werden. Die Konzentration wird in der internationalen Einheit kU/I (kilo IgE-Units pro Liter) dargestellt. Für die Interpretation des Ergebnisses steht z.B. das in der Allergiediagnostik weit verbreitete RAST-System (Radio-Allergo-Sorbent-Test), entwickelt von der Firma Pharmacia (heute Thermo Scientific), als Referenzsysteme zur Verfügung (Buhl et al., "Allergologie - Handbuch", Hrsg. von K. Mohr. Stuttgart: Schattauer.2011). Das EUROLINE-Scan-Programm rechnet automatisch die Bandenintensitäten des Eurolines in EAST (Enzym-Allergo-Sorbent-Test)-Klassen um, die in

Bezug auf die Konzentrationsabstufungen dem RAST- System entsprechen. Die Einteilung der EAST(Enzym-Allergo-Sorbent-Test)-Klassen ist in Tab. 1 dargestellt:

**Tab. 1: EAST-Klassen**

| **EAST-Klasse** | **Konzentration [kU/I]** |
|---|---|
| 0 | < 0,35 |
| 1 | 0.35 - 0,7 |
| 2 | 0,7 - 3,5 |
| 3 | 3,5 - 17,5 |
| 4 | 17,5 - 50 |
| 5 | 50 - 100 |
| 6 | > 100 |
| **EAST-Klassen** | |

Bei den für den Euroline mit Cor a 12 eingesetzten Patientenseren handelte es sich um vorcharakterisierte Haselnuss-Allergiker-Seren. Bei der Negativkontrolle wurde kein Serum mit inkubiert.

## Patentansprüche

1. Polypeptid umfassend ein antigenes Fragment mit einer Sequenz von wenigstens sieben Aminosäuren aus einer hydrophilen Domäne eines Ölkörper-assoziierten Proteins,
wobei das Polypeptid keine oder eine verkürzte hydrophobe Domäne des Ölkörperassoziierten Proteins aufweist,
und wobei das Ölkörper-assoziierte Protein aus der Gruppe ausgewählt ist, die Oleosin, Caleosin und Steroleosin umfasst.

2. Polypeptid nach Anspruch 1, umfassend
a) ein antigenes Fragment von wenigstens sieben Aminosäuren aus einer N-terminalen hydrophilen Domäne eines Ölkörper-assoziierten Proteins und
b) ein antigenes Fragment von wenigstens sieben Aminosäuren aus einer C-terminalen hydrophilen Domäne eines Ölkörper-assoziierten Proteins,
wobei a) und b) aneinander fusioniert oder durch einen Linker verbunden sind,
und wobei das Ölkörper-assoziierte Protein aus der Gruppe ausgewählt ist, die Oleosin und Caleosin umfasst.

3. Polypeptid gemäß Anspruch 1,
wobei das Ölkörper-assoziierte Protein ein Oleosin ist.

4. Polypeptid nach einem der Ansprüche 1 oder 2, wobei es sich bei a) um eine Sequenz aus SEQ ID NO1 und Varianten davon und bei b) um eine Sequenz aus SEQ ID NO2 und Varianten davon handelt.

5. Polypeptid nach einem der Ansprüche 1 oder 2, wobei es sich bei a) um eine Sequenz aus SEQ ID NO3 und Varianten davon und bei b) um eine Sequenz aus SEQ ID NO4 und Varianten davon handelt.

6. Polypeptid nach einem der Ansprüche 1 bis 5, wobei das Polypeptid immobilisiert ist, bevorzugt an einem festen Träger, noch bevorzugter einem Mikrokügelchen.

7. Pharmazeutische Zusammensetzung oder Impfstoff umfassend das Polypeptid nach einem der Ansprüche 1 bis 6.

8. Komplex umfassend das Polypeptid nach einem der Ansprüche 1 bis 6 sowie einen daran gebundenen Antikörper, bevorzugt der IgE- Klasse.

9. Medizinische oder diagnostische Vorrichtung, umfassend das Polypeptid nach Anspruch 1 bis 4.

10. Medizinische oder diagnostische Vorrichtung nach Anspruch 8, wobei es sich um einen Teststreifen, einen Biochip oder ein Elektrophoresegel handelt.

11. Nucleinsäure kodierend für das Polypeptid nach einem der Ansprüche 1 bis 4 oder Zelle umfassend diese Nucleinsäure.

12. Testkit umfassend das Polypeptid nach einem der Ansprüche 1 bis 4, optional zusätzlich ein Mittel zum Nachweis des Komplexes nach Anspruch 8.

13. Verfahren umfassend den Schritt Nachweisen eines Antikörpers, der gegen das Polypeptid nach einem der Ansprüche 1 bis 4 bindet, in einer Probe eines Säugetiers, bevorzugt eines Menschen, die Antikörper enthält, bevorzugt der IgE- Klasse.

14. Verfahren nach Anspruch 12, wobei das Säugetier wenigstens ein Symptom einer Allergie aufweist und/oder der Verdacht besteht, dass das Säugetier unter einer Allergie leidet, bevorzugt gegen Nüsse.

15. Verfahren nach einem der Ansprüche 12 bis 13, umfassend die Schritte
a) Bereitstellen einer Probe eines Säugetiers,
b) Kontaktieren der Probe mit dem Polypeptid nach einem der Ansprüche 1 bis 5 oder der medizinischen Vorrichtung gemäß einem der Ansprüche 7 oder 8 unter Bedingungen, die mit der Bildung eines Komplexes zwischen dem Polypeptid und einem Antikörper kompatibel sind, und
c) Nachweisen eines in Schritt b) gebildeten Komplexes.
